# EUROPEAN PATENT SPECIFICATION

(11) **EP 2 327 355 B1**
(45) Date of publication and mention of the grant of the patent: **26.07.2017**
(21) Application number: 09811469.7
(22) Date of filing: 31.08.2009
(51) Int. Cl.: A61B 1/00, A61B 1/04, A61B 5/06, A61B 5/07, H04B 13/00, A61B 5/00, A61B 34/00

(54) **CAPSULE GUIDANCE SYSTEM**
KAPSELFÜHRUNGSSYSTEM
SYSTÈME DE GUIDAGE DE CAPSULE

(30) Priority: 02.09.2008 JP 2008225148
(43) Date of publication of application: 01.06.2011
(73) Proprietor: Olympus Corporation, Tokyo 192-8507 (JP)
(72) Inventor: UCHIYAMA, Akio, Hachioji-shi, Tokyo 192-8507 (JP); KIMURA, Atsushi, Hachioji-shi, Tokyo 192-8507 (JP); MINAI, Tetsuo, Hachioji-shi, Tokyo 192-8507 (JP); TAKIZAWA, Hironobu, Hachioji-shi, Tokyo 192-8507 (JP); MORI, Takeshi, Hachioji-shi, Tokyo 192-8507 (JP)
(74) Representative: Gunzelmann, Rainer
(86) International application number: PCT/JP2009/065181
(87) International publication number: WO 2010/026943

(56) References cited:
- WO-A1-2004/068748
- WO-A1-2006/097423
- WO-A1-2008/026549
- WO-A2-2008/016194
- JP-A- 8 084 779
- JP-A- 2008 035 968
- JP-A- 2008 054 774
- JP-A- 2008 119 053
- JP-A- 2008 132 050
- JP-T- 2006 513 001
- JP-T- 2006 513 670
- US-A1- 2008 125 623

## Description

### Field

The present invention relates to a capsule guidance system which performs human body communication with a capsule medical device inserted inside a human body such as a patient, detects the position of the capsule medical device, and guides the capsule medical device inside the human body.

### Background

In recent years, in the field of endoscope, a swallowing-type capsule endoscope (an example of a capsule medical device) appears. The capsule endoscope has an imaging function and a wireless communication function. The capsule endoscope has a function in which, after the capsule endoscope is swallowed from a mouth of a patient for an observation (examination), the capsule endoscope moves inside the body, for example, insides of organs such as a stomach and a small intestine following a peristaltic motion inside the patient and sequentially captures images of the insides of the organs until the capsule endoscope is naturally discharged from the human body.

However, the capsule endoscope communicates with the outside of the human body by using a wireless communication function, so that the power consumption is large, the operation time is short, and the volume occupied by a primary battery is large. This causes a problem that downsizing and enhancement of the capsule endoscope are limited. Therefore, in recent years, a human body communication system appears which performs communication (in other words, human body communication) in which a capsule endoscope inside a human body (inside an organ) and a receiving device outside the human body communicate with each other using the human body as a communication medium.

In the human body communication system described above, an electric current is generated by a voltage difference between transmitting electrodes formed on the surface of the capsule endoscope. When the electric current flows through the human body, a voltage is induced between two receiving electrodes attached on the human body, and a receiving device outside the human body receives data from the capsule endoscope by using the induced voltage. The capsule endoscope using the human body communication described above can transmit data using a low frequency signal of about 10 MHz without requiring a high-frequency signal of several hundred MHz, so that the power consumption can be extremely reduced (see Patent Literatures 1 and 2).

On the other hand, there is a magnetic guidance system in which a magnet is provided in a capsule endoscope, an external rotating magnetic field is applied to the capsule endoscope to rotate the capsule endoscope, and the capsule endoscope in a subject is guided to a desired position by the rotation to perform an examination (see Patent Literatures 3 and 4).

When combining together the human body communication system and the magnetic guidance system, it is possible to realize a capsule guidance system which receives data from the capsule endoscope inside an organ of a subject by using voltages detected by a plurality of receiving electrode pairs deposed on the body surface of the subject to perform the human body communication, detects the position of the capsule endoscope inside the organ, and guides the capsule endoscope inside the organ on the basis of the detected position.

### Citation List

### Patent Literature

Patent Literature 1: Japanese National Publication of International Patent Application No. 2006-513001
Patent Literature 2: Japanese National Publication of International Patent Application No. 2006-513670
Patent Literature 3: Japanese Laid-open Patent Publication No. 2004-255174
Patent Literature 4: Japanese Laid-open Patent Publication No. 2005-304638

### Summary

### Technical Problem

However, in the above-mentioned conventional capsule guidance system, when guiding the capsule endoscope inserted into an organ of the subject, the external rotating magnetic field applied to the capsule endoscope may rapidly change over time, so that there is a risk that an eddy current is generated on the body surface of the subject due to the rapid change over time of the external rotating magnetic field, and an unnecessary voltage caused by the generated eddy current is detected by the receiving electrode pairs for the human body communication. As a result, there is a problem that it is difficult to detect the position of the capsule endoscope in the subject due to the eddy current.

The present invention is made in view of the above situation, and an object of the invention is to provide a capsule guidance system which can detect the position of the capsule endoscope in a subject without being disturbed by the eddy current generated on the body surface when the capsule endoscope in the subject is magnetically guided.

Document WO 2008/026549 A1 discloses a capsule guiding system and a capsule guiding method which allow a precise detection of at least one of a position and a direction, inside a subject body, of a capsule endoscope using human body communication, and a precise guidance of the capsule endoscope.

### Solution to Problem

To solve the problem described above and achieve the object, a capsule guidance system according to the present invention includes the features of claim 1. Further embodiments are defined in the dependent claims 2-8.

### Advantageous Effects of Invention

The capsule guidance system according to the present invention is configured to apply a magnetic field to a capsule medical device introduced inside an organ of a subject to guide the capsule medical device, perform human body communication with the capsule medical device to receive an electrical signal transmitted from the capsule medical device, and when an eddy current is generated in the subject due to a change of the magnetic field, extract the electrical signal transmitted from the capsule medical device by eliminating a signal component of the eddy current to calculate the position of the capsule medical device on the basis of the electrical signal transmitted from the capsule medical device. Therefore, when magnetically guiding the capsule medical device in the subject, the capsule guidance system can eliminate a signal component of the eddy current caused by the magnetic field from the electrical signal detected by electrode pads on the body surface of the subject. As a result, the capsule guidance system has an effect that the position of the capsule medical device inside the subject can be detected without being disturbed by the eddy current.

### Brief Description of Drawings

FIG. 1 is a block diagram schematically showing a configuration example of a capsule guidance system according to a first embodiment of the present invention.
FIG. 2 is a schematic diagram showing a configuration example of a capsule endoscope of the capsule guidance system according to the first embodiment of the invention.
FIG. 3 is a schematic diagram showing a configuration example of a magnetic field generator of the capsule guidance system according to the first embodiment of the invention.
FIG. 4 is a flowchart illustrating a processing procedure of a control unit that controls operation timings of an image processing unit and a position calculator to eliminate a signal component of eddy current.
FIG. 5 is a diagram illustrating change over time of the strength of the magnetic field applied from the magnetic field generator to the capsule endoscope inside a subject.
FIG. 6 is a block diagram schematically showing a configuration example of a capsule guidance system according to a second embodiment of the invention.
FIG. 7 is a flowchart illustrating a processing procedure of a control unit to eliminate a signal component of eddy current.
FIG. 8 is a block diagram schematically showing a configuration example of a capsule guidance system according to a third embodiment of the invention.
FIG. 9 is a block diagram schematically showing a configuration example of a capsule guidance system according to a fourth embodiment of the invention.
FIG. 10 is a block diagram schematically showing a configuration example of a receiving unit of the capsule guidance system according to the fourth embodiment of the invention.

### Description of Embodiments

Hereinafter, a capsule guidance system which is a best mode for carrying out the present invention will be described. Although, in the description below, a capsule endoscope which captures images of the inside of an organ of a subject (hereinafter, sometimes referred to as in-vivo image) is illustrated as an example of a capsule medical device of the capsule guidance system according to the invention, the invention is not limited to this embodiment.

### First Embodiment

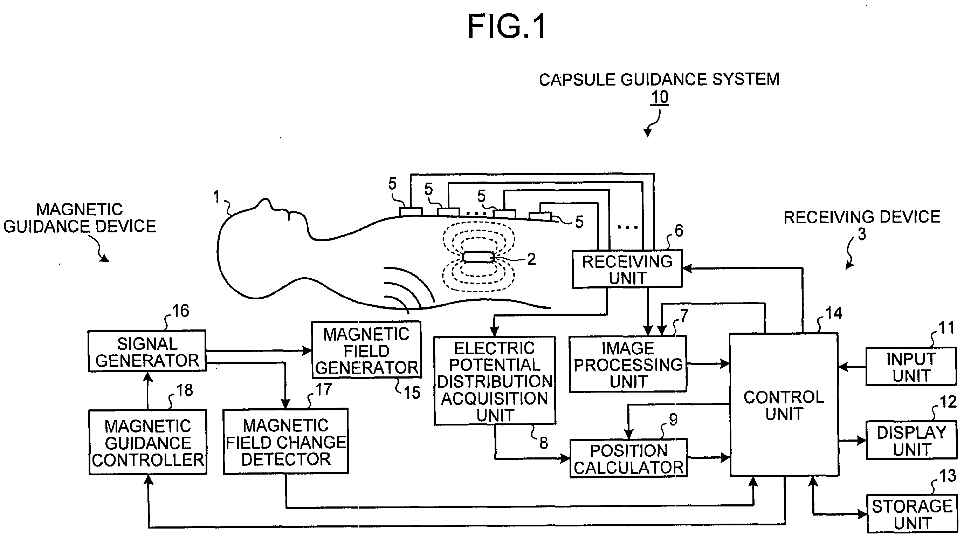

FIG. 1 is a block diagram schematically showing a configuration example of a capsule guidance system according to a first embodiment of the present invention. As shown in FIG. 1, a capsule guidance system 10 according to the first embodiment includes a capsule endoscope 2 inserted into an organ of a subject 1 such as a patient, a receiving device 3 for receiving data transmitted from the capsule endoscope 2 inside the subject 1, and a magnetic guidance device 4 for guiding the capsule endoscope 2 inside the subject 1 by magnetic force.

The capsule endoscope 2 is an example of the capsule medical device inserted into an organ of the subject 1, and has an imaging function and a human body communication function. Specifically, when the capsule endoscope 2 is inserted into an organ of the subject 1, the capsule endoscope 2 captures in-vivo images of the subject 1 while moving inside the organ of the subject 1, and performs the human body communication for transmitting the captured in-vivo images to the receiving device 3 outside the subject 1. In this case, the capsule endoscope 2 flows an electric current to the subject 1 to transmit an in-vivo image of the subject 1, and forms an electric potential distribution in the body of the subject 1 or on the body surface of the subject 1. As a result, the capsule endoscope 2 transmits an electrical signal corresponding to the in-vivo image of the subject 1 to the receiving device 3 using the subject 1 (that is, a human body) as a communication medium. The capsule endoscope 2 repeatedly performs the human body communication with the receiving device 3 every time an in-vivo image of the subject 1 is captured, and sequentially transmits electrical signals corresponding to the in-vivo image of the subject 1 to the receiving device 3.

The receiving device 3 has a human body communication function for performing human body communication with the capsule endoscope 2 inside an organ using the subject 1 as a communication medium and a position detection function for detecting the position of the capsule endoscope 2 inside the subject 1. As shown in FIG. 1, the receiving device 3 includes a plurality of electrode pads 5 disposed on the body surface of the subject 1, a receiving unit 6 for receiving the electrical signal from the capsule endoscope 2 via the plurality of electrode pads 5, an image processing unit 7 for generating an in-vivo image of the subject 1 corresponding to the electrical signal received by the receiving unit 6, an electric potential distribution acquisition unit 8 for acquiring the electric potential distribution formed on the body surface of the subject 1, and a position calculator 9 for calculating the position of the capsule endoscope 2 inside the subject 1. The receiving device 3 also includes an input unit 11 for inputting various information, a display unit 12 for displaying various information such as an in-vivo image of the subject 1, a storage unit 13 for storing in-vivo images of the subject 1, the position information of the capsule endoscope 2, and the like, and a control unit 14 for controlling each unit of the receiving device 3.

The plurality of electrode pads 5 are to detect an electrical signal from the capsule endoscope 2 which is transmitted via the subject 1 (that is, a human body) used as a communication medium. Specifically, the plurality of electrode pads 5, each of which has an electrode pair, are dispersed and disposed at predetermined positions on the body surface of the subject 1 in which the capsule endoscope 2 is inserted into an organ. A voltage is induced in each of the electrode pairs of the plurality of electrode pads 5 which are dispersed and disposed on the body surface of the subject 1 when the capsule endoscope 2 transmits an electrical signal using the subject 1 as a communication medium. In this case, each of the plurality of electrode pads 5 detects the voltage induced in each electrode pair in such a way as an electrical signal, and transmits the detected electrical signal to the receiving unit 6. The electrical signals detected by the electrode pads 5 include at least the electrical signal from the capsule endoscope 2 (specifically, the electrical signal transmitted/received by the human body communication between the capsule endoscope 2 inside the subject 1 and the receiving device 3).

The receiving unit 6 receives the electrical signals detected by the plurality of electrode pads 5 described above. Specifically, the receiving unit 6 is connected to the plurality of electrode pads 5, and receives the voltages induced in each electrode pair of the plurality of electrode pads 5 as electrical signals. Here, the electrical signals received by the receiving unit 6 include at least the electrical signal from the capsule endoscope 2. The receiving unit 6 selects an electrical signal corresponding to a highest voltage among the voltages detected by the plurality of electrode pads 5, and performs demodulation processing or the like on the selected electrical signal to demodulate the electrical signal into an image signal. The image signal includes an in-vivo image of the subject 1 which is captured by the capsule endoscope 2. The receiving unit 6 transmits the image signal obtained by the demodulation processing as described above to the image processing unit 7.

The receiving unit 6 also transmits voltage values at the positions of each of the plurality of electrode pads 5 disposed on the body surface of the subject 1 to the electric potential distribution acquisition unit 8. Specifically, the receiving unit 6 receives each electrical signal detected by each of the plurality of electrode pads 5, and transmits each of the received electrical signals to the electric potential distribution acquisition unit 8. Based on this, the receiving unit 6 transmits voltage values of the electrical signals detected by each of the plurality of electrode pads 5, in other words, voltage values induced in each of the electrode pairs of the plurality of electrode pads 5 to the electric potential distribution acquisition unit 8. In this way, the receiving unit 6 achieves voltage transmission processing for transmitting voltage values at the positions of each of the plurality of electrode pads 5 to the electric potential distribution acquisition unit 8.

The receiving unit 6 is controlled by the control unit 14 to repeatedly perform the demodulation processing of the image signal and the voltage transmission processing described above every time the capsule endoscope 2 performs the human body communication for transmitting an in-vivo image of the subject 1.

The image processing unit 7 functions as a data acquisition means for acquiring an in-vivo image of the subject 1 captured by the capsule endoscope 2 (an example of data from the capsule medical device) on the basis of the electrical signal transmitted from the capsule endoscope 2 by the human body communication. Specifically, the image processing unit 7 acquires the image signal demodulated by the receiving unit 6, and performs predetermined image processing on the acquired image signal. Here, as described above, the image signal acquired from the receiving unit 6 includes an in-vivo image of the subject 1 captured by the capsule endoscope 2. The image processing unit 7 generates (reconstructs) the in-vivo image of the subject 1 on the basis of the image signal, and transmits the generated in-vivo image of the subject 1 to the control unit 14. The image generation processing timing of the image processing unit 7 is controlled by the control unit 14.

The electric potential distribution acquisition unit 8 acquires the electric potential distribution formed on the body surface of the subject 1. Specifically, the electric potential distribution acquisition unit 8 acquires the voltage values at the positions of each of the plurality of electrode pads 5, which are transmitted from the receiving unit 6. Here, the electric potential distribution acquisition unit 8 previously knows the positions of each of the plurality of electrode pads 5 disposed on the body surface of the subject 1. Every time the receiving unit 6 receives the electrical signals detected by the plurality of electrode pads 5, the electric potential distribution acquisition unit 8 grasps the relationship between the positions of each of the plurality of electrode pads 5 on the body surface of the subject 1 and the voltage values of each of the plurality of electrode pads 5 on the basis of the voltage values transmitted from the receiving unit 6, and acquires the electric potential distribution formed on the body surface of the subject 1 (hereinafter referred to as electric potential distribution on the subject 1) on the basis of the relationship. The electric potential distribution acquisition unit 8 understands the electric potential distribution formed on the body surface of the subject 1 using the electrical signals from the capsule endoscope 2 when an eddy current is not generated in the subject 1. The electric potential distribution acquisition unit 8 transmits the acquired electric potential distribution on the subject 1 to the position calculator 9.

The position calculator 9 functions as a position calculation means for calculating the position of the capsule endoscope 2 inside the subject 1 on the basis of the electrical signals from the capsule endoscope 2 detected by the plurality of electrode pads 5. Specifically, the position calculator 9 obtains the electric potential distribution on the subject 1 transmitted from the electric potential distribution acquisition unit 8, and calculates the position of the capsule endoscope 2 inside the subject 1 on the basis of the obtained electric potential distribution on the subject 1. For example, the position calculator 9 previously has a voltage value of the capsule endoscope 2 that is a voltage source of the electric potential distribution on the subject 1 (specifically, a voltage value generated between transmitting electrodes of the capsule endoscope 2 when the human body communication is performed) as known information. Also, the position calculator 9 previously has an error function for calculating an error between estimated values of voltages induced in each electrode pad 5 when the capsule endoscope 2 is located in an assumed position inside the subject 1 which is temporarily set and the electric potential distribution on the subject 1 (that is, actual measured values of voltages induced in each electrode pad 5). The position calculator 9 repeatedly calculates the error between the estimated values of voltages of each electrode pad 5 and the electric potential distribution on the subject 1 using the error function, and calculates an assumed position where the error is smallest inside the subject 1 as the position of the capsule endoscope 2 inside the subject 1. The position calculator 9 transmits the position information of the capsule endoscope 2 calculated in this way to the control unit 14. The position calculation processing timing of the position calculator 9 is controlled by the control unit 14.

The position calculator 9 may calculate the position of the capsule endoscope 2 inside the subject 1 not only by the position calculation processing of the capsule endoscope 2 using the error function as described above, but also (for example, on the basis of trigonometry) on the basis of calculated distances obtained by calculating distances between the capsule endoscope 2 and a plurality of electrode pads 5 on the basis of the voltage value of the capsule endoscope 2 which is the voltage source of the electric potential distribution on the subject 1, the electric potential distribution on the subject 1 (actual measured values of voltages induced in each electrode pad 5), and an impedance of the subject 1 which is the communication medium.

The input unit 11 is realized by using input devices such as a keyboard and a mouse, and inputs various information into the control unit 14 according to an input operation of a user such as a doctor or a nurse. The various information inputted from the input unit 11 to the control unit 14 includes, for example, instruction information for instructing the control unit 14 (including instruction information for operating the magnetic guidance device 4), patient information of the subject 1, and examination information of the subject 1.

The patient information of the subject 1 is identification information for identifying the subject 1, and includes, for example, patient name, patient ID, birth date, sex, and age of the subject 1. The examination information of the subject 1 is identification information for identifying a capsule endoscope examination performed on the subject 1 (examination for observing the inside of an organ by inserting the capsule endoscope 2 into the organ), and includes, for example, examination ID, and examination date.

The display unit 12 is realized by using various types of displays such as a CRT display or a liquid crystal display, and displays various information which is instructed to be displayed by the control unit 14. Specifically, the display unit 12 displays the in-vivo images of the subject 1 captured by the capsule endoscope 2, the patient information of the subject 1, the examination information of the subject 1, the position information of the capsule endoscope 2 inside the subject 1, and the like.

The storage unit 13 is realized by using various types of storage media, which stores data in a rewritable manner, such as a RAM, an EEPROM, a flash memory, or a hard disk. The storage unit 13 stores various data instructed to be stored by the control unit 14, and transmits data instructed to be read from the stored various data by the control unit 14 to the control unit 14. The storage unit 13 stores the in-vivo images of the subject 1 generated by the image processing unit 7, the patient information and the examination information of the subject 1 inputted by the input unit 11, and the position information of the capsule endoscope 2 calculated by the position calculator 9 on the basis of the control of the control unit 14.

The storage unit 13 may be realized by using a drive into which a portable recording medium such as a flexible disk (FD), a compact disk (CD), or a DVD (Digital Versatile Disk) can be attachably and detachably inserted and which writes or reads various data to or from the inserted portable recording medium.

The control unit 14 controls operations of each unit (receiving unit 6, image processing unit 7, electric potential distribution acquisition unit 8, position calculator 9, input unit 11, display unit 12, and storage unit 13) of the receiving device 3, and controls input/output of signals between the units. Specifically, the control unit 14 starts the operation of the receiving unit 6 on the basis of instruction information inputted from the input unit 11, and controls the receiving unit 6 to repeatedly perform the demodulation processing of the image signal and the voltage transmission processing described above every time the capsule endoscope 2 performs the human body communication. The control unit 14 causes the storage unit 13 to store the in-vivo images of the subject 1 generated by the image processing unit 7 and the position information of the capsule endoscope 2 calculated by the position calculator 9, and also causes the display unit 12 to display the in-vivo images and the position information. The control of the control unit 14 is performed on the basis of the instruction information inputted from the input unit 11.

The control unit 14 controls the image generation processing timing of the image processing unit 7 and the position calculation processing timing of the position calculator 9 so that an in-vivo image of the subject 1 can be generated and the position of the capsule endoscope 2 can be calculated without being affected by eddy current generated on the body surface of the subject 1. Specifically, the control unit 14 controls the operation timing of the image processing unit 7 and the operation timing of the position calculator 9 so that, when an eddy current is generated on the body surface of the subject 1, both the image generation processing of the image processing unit 7 and the position calculation processing of the position calculator 9 are stopped. The eddy current is generated due to a change in magnetic field caused by the magnetic guidance device 4 to guide the capsule endoscope 2 inside the subject 1. Therefore, the control unit 14 obtains the time rate of change of the magnetic field detected by a magnetic field change detector 17 of the magnetic guidance device 4 described below, and controls the image generation processing timing of the image processing unit 7 and the position calculation processing timing of the position calculator 9 on the basis of the obtained time rate of change of the magnetic field.

On the other hand, the magnetic guidance device 4 functions as a magnetic guidance means for magnetically guiding the capsule endoscope 2 inserted into an organ of the subject 1. Specifically, the magnetic guidance device 4 applies a magnetic field (for example, a rotating magnetic field) capable of guiding the capsule endoscope 2 inside the subject 1 in a desired direction to the capsule endoscope 2, and moves the capsule endoscope 2 inside the subject 1 to a desired position by the magnetic field. As shown in FIG. 1, the magnetic guidance device 4 includes a magnetic field generator 15 for generating a magnetic field capable of guiding the capsule endoscope 2 inside the subject 1, a signal generator 16 for generating an AC signal supplied to the magnetic field generator 15 to generate the magnetic field, the magnetic field change detector 17 for detecting the time rate of change of the magnetic field generated by the magnetic field generator 15, and a magnetic guidance controller 18 for controlling the guidance of the capsule endoscope 2 by the magnetic field generated by the magnetic field generator 15.

The magnetic field generator 15 is realized by combining electromagnets such as Helmholtz coils, and generates a magnetic field capable of guiding the capsule endoscope 2 inside the subject 1 to a desired position when the electric current is supplied from the signal generator 16. Specifically, the magnetic field generator 15 has a configuration capable of generating a magnetic field in each of the X axis direction, the Y axis direction, and the Z axis direction in a predetermined three-axis coordinate system, and forms a three-dimensional rotating magnetic field (an example of a magnetic field capable of guiding the capsule endoscope 2 inside the subject 1) in the position of the capsule endoscope 2 inside the subject 1 by changing the strength of the magnetic fields generated in each of the X, Y, and Z axis directions. The magnetic field generator 15 functions as a magnetic guidance means for guiding the capsule endoscope 2 to a desired position in the subject 1 by applying a magnetic field (for example, three-dimensional rotating magnetic field) to the capsule endoscope 2 inside the subject 1.

The signal generator 16 functions to supply an electric current necessary to generate a magnetic field capable of guiding the capsule endoscope 2 inside the subject 1 to the magnetic field generator 15. Specifically, the signal generator 16 generates an AC signal necessary to generate a magnetic field (for example, three-dimensional rotating magnetic field) capable of guiding the capsule endoscope 2 to a desired position inside the subject 1 on the basis of the control of the magnetic guidance controller 18, and transmits the generated AC signal to the magnetic field generator 15. Based on this, the signal generator 16 supplies an electric current necessary to generate a magnetic field capable of guiding the capsule endoscope 2 to the magnetic field generator 15. Every time the signal generator 16 generates an AC signal in this way, the signal generator 16 also transmits the generated AC signal to the magnetic field change detector 17.

The magnetic field change detector 17 functions as a magnetic field change detecting means for detecting a change of magnetic field generated by the magnetic field generator 15 to guide the capsule endoscope 2 inside the subject 1. Specifically, the magnetic field change detector 17 sequentially obtains, from the signal generator 16, the same AC signals as those supplied to the magnetic field generator 15 to guide the capsule endoscope 2 inside the subject 1, and monitors change over time of the value of the current sequentially supplied from the signal generator 16 to the magnetic field generator 15 on the basis of the obtained AC signals. The magnetic field change detector 17 detects the time rate of change of the magnetic field applied to the capsule endoscope 2 by the magnetic field generator 15 on the basis of the monitoring result of the change over time of the current value. The magnetic field change detector 17 transmits the time rate of change of the magnetic field detected in this way to the control unit 14 of the receiving device 3.

The magnetic guidance controller 18 controls the current value of the AC signal supplied from the signal generator 16 to the magnetic field generator 15 to generate a magnetic field capable of guiding the capsule endoscope 2 to a desired position inside the subject 1, and controls the magnetic guidance of the capsule endoscope 2 inside the subject 1 through the control of the current value (that is, a control of the amount of current supplied to the magnetic field generator 15). Specifically, the magnetic guidance controller 18 is connected to the control unit 14 of the receiving device 3 described above, and obtains operational instruction information related to the magnetic guidance from the control unit 14. The operational instruction information related to the magnetic guidance is instruction information for operating the magnetic guidance device 4 to guide the capsule endoscope 2 inside the subject 1 to a desired position, and inputted in the magnetic guidance controller 18 from the input unit 11 via the control unit 14. Every time the above-described position calculator 9 calculates the position of the capsule endoscope 2, the magnetic guidance controller 18 obtains the position information of the capsule endoscope 2 from the control unit 14. The magnetic guidance controller 18 grasps the current position of the capsule endoscope 2 inside the subject 1 on the basis of the position information of the capsule endoscope 2 calculated by the position calculator 9. The magnetic guidance controller 18 then obtains the operational instruction information related to the magnetic guidance from the input unit 11 via the control unit 14, and controls the amount of current supplied to the magnetic field generator 15 to move the capsule endoscope 2 to the position inside the subject 1 instructed by the instruction information.

Here, the above-described magnetic field change detector 17 and the control unit 14 of the receiving device 3 form an eliminating means for eliminating signal components of eddy current from electrical signals detected by the plurality of electrode pads,5. Specifically, as described above, the magnetic field change detector 17 detects the time rate of change of the magnetic field applied to the capsule endoscope 2 inside the subject 1, and transmits the detected time rate of change of the magnetic field to the control unit 14 of the receiving device 3. The control unit 14 is connected to the magnetic field change detector 17, and controls the image generation processing timing of the image processing unit 7 and the position calculation processing timing of the position calculator 9 on the basis of the time rate of change of the magnetic field transmitted from the magnetic field change detector 17. In this case, the control unit 14 stops both the image generation processing of the image processing unit 7 and the position calculation processing of the position calculator 9 in a state in which an eddy current is generated in the subject 1 due to a rapid change of the strength of the magnetic field applied to the capsule endoscope 2. As a result, the control unit 14 eliminates an electrical signal including a signal component of eddy current as a noise component (hereinafter referred to as noise-containing signal) from the electrical signals received by the receiving unit 6 (specifically, the electrical signals detected by the plurality of electrode pads 5). In this way, the signal component of eddy current is eliminated.

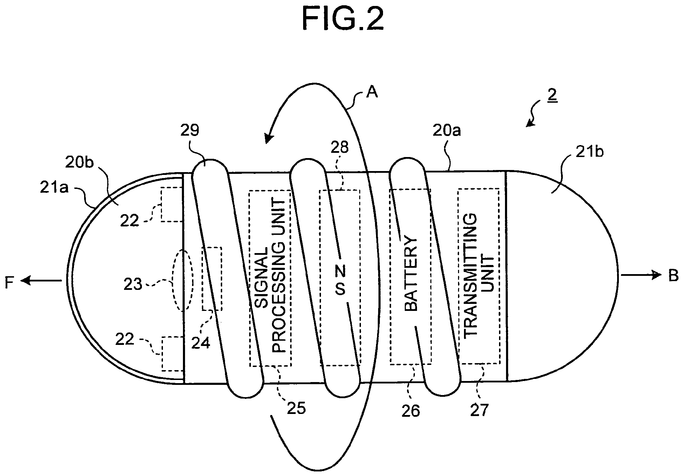

Next, a configuration of the capsule endoscope 2 that can be guided by the magnetic field generated by the magnetic field generator 15 described above will be described. FIG. 2 is a schematic diagram showing a configuration example of the capsule endoscope 2 of the capsule guidance system according to the first embodiment of the invention. As shown in FIG. 2, the capsule endoscope 2 has a capsule-shaped structure in which one end of an opaque tubular housing 20a has an opaque dome shape and the other end is closed by a transparent dome-shaped housing 20b. In the tubular housing 20a and the dome-shaped housing 20b, an illumination unit 22 realized by an LED or the like, a condenser lens 23, and an imaging element 24 are provided on the side of the dome-shaped housing 20b, and images of objects around the dome-shaped housing 20b are captured. An imaging signal outputted from the imaging element 24 is processed by a signal processing unit 25, outputted from a transmitting unit 27 by transmitting electrodes 21a and 21b described below as an image signal, and transmitted to the electrode pads 5 through the subject 1 (that is, a human body).

The transmitting electrodes 21a and 21b for the human body communication are formed respectively on the surface of the dome-shaped housing 20b and the surface of the dome opposite to the dome-shaped housing 20b. The transmitting electrode 21a formed on the surface of the dome-shaped housing 20b is a transparent electrode realized by ITO or the like. The transmitting electrodes 21a and 21b are metal which has good corrosion resistance property and is harmless to the human body. For example, the transmitting electrode 21b is realized by SUS316L or gold. Further, the transmitting electrodes 21a and 21b are electrically connected to the inside of the human body through body fluids or the like.

A battery 26 and a magnet 28 are disposed in the center of the capsule endoscope 2. As shown in FIG. 2, the magnetic poles of the magnet 28 are arranged in a direction perpendicular to the longitudinal direction of the capsule endoscope 2. When a rotating magnetic field is applied around the axis of the capsule endoscope 2, the magnet 28 is attracted to the rotating magnetic field and rotates around the axis like a rotor of a motor. In this way, the magnet 28 rotates around the axis, and thereby the capsule endoscope 2 rotates.

A helical protrusion 29 is formed around the tubular portion of the capsule endoscope 2. When the capsule endoscope 2 rotates by the rotation of the magnet 28, the helical protrusion 29 fits into the wall of the digestive tract in the body like a screw. The capsule endoscope 2 moves in the axis direction like a screw by the screw-like contact between the helical protrusion 29 and the wall of the digestive tract. For example, in FIG. 2, when the capsule endoscope 2 rotates in the A direction around the axis, the capsule endoscope 2 proceeds in the F direction, and when the capsule endoscope 2 rotates in the direction opposite to the A direction around the axis, the capsule endoscope 2 goes backward in the B direction. When the magnetic field generator 15 applies the rotation magnetic field for rotating the capsule endoscope 2 to the capsule endoscope 2 in this way, the capsule endoscope 2 can move in the subject 1.

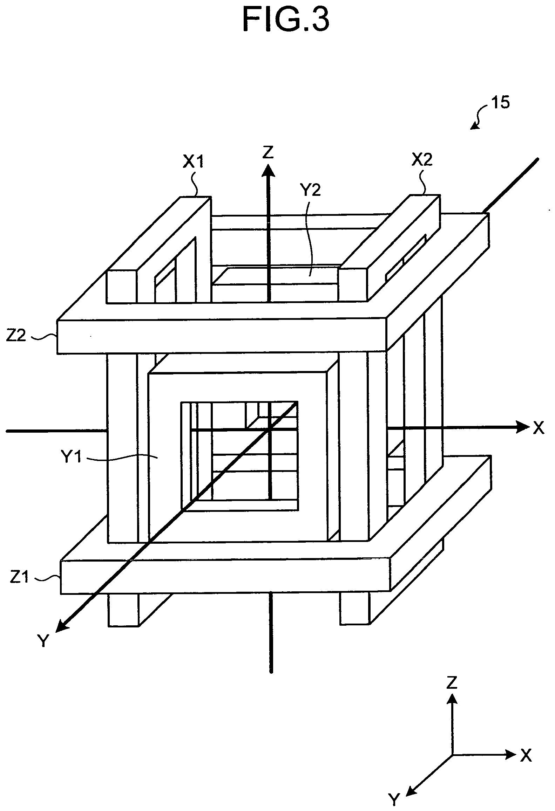

Next, a configuration of the magnetic field generator 15 that generates the rotating magnetic field capable of guiding the capsule endoscope 2 will be described. FIG. 3 is a schematic diagram showing a configuration example of the magnetic field generator 15 of the capsule guidance system according to the first embodiment of the invention. As shown in FIG. 3, the magnetic field generator 15 includes electromagnets in which a coil is wound around a member having a high dielectric constant, such as a ferromagnetic body, and has a configuration in which pairs of electromagnets (for example, Helmholtz coils) are combined so that the subject 1 is sandwiched from three directions of X, Y, and Z. Specifically, the magnetic field generator 15 is formed by combining a pair of electromagnets X1 and X2 generating a magnetic field in the X direction, a pair of electromagnets Y1 and Y2 generating a magnetic field in the Y direction, and a pair of electromagnets Z1 and Z2 generating a magnetic field in the Z direction. The magnetic field generator 15 can form a three-dimensional rotating magnetic field over the capsule endoscope 2 inside the subject 1 by controlling the strengths of the magnetic fields generated in the X, Y, and Z axis directions on the basis of the AC signal supplied from the signal generator 16. The formation of the rotating magnetic field is performed by the magnetic guidance controller 18 controlling the amount of current supplied to the electromagnets in the X, Y, and Z axis directions on the basis of the operation instruction of the instruction information (operational instruction information related to the magnetic guidance) inputted from the input unit 11 via the control unit 14.

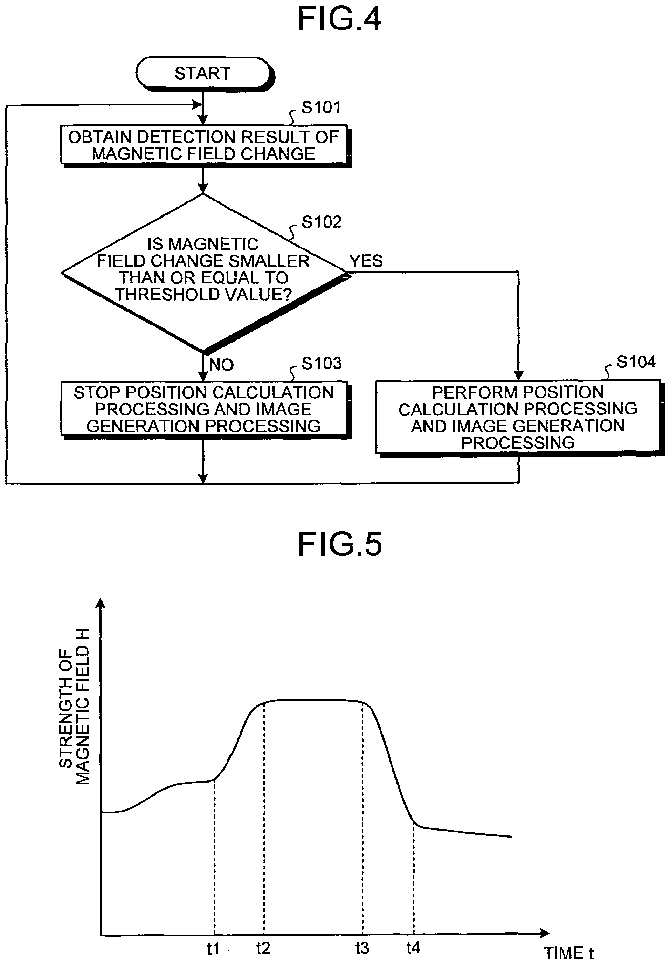

Next, an operation of the control unit 14 which forms an eliminating means for eliminating a signal component of eddy current will be described. FIG. 4 is a flowchart illustrating a processing procedure of the control unit 14 that controls operation timings of the image processing unit 7 and the position calculator 9 to eliminate a signal component of eddy current. When an eddy current is generated on the body surface of the subject 1, the control unit 14 controls operation timings of the image processing unit 7 and the position calculator 9 to eliminate a signal component of the eddy current from the electrical signals detected by the plurality of electrode pads 5 described above.

That is, as shown in FIG. 4, the control unit 14 obtains a detection result of the change of the magnetic field detected by the magnetic field change detector 17 (step S101). Specifically, as described above, the magnetic field change detector 17 monitors change over time of the value of the current sequentially supplied from the signal generator 16 to the magnetic field generator 15, and detects the time rate of change of the magnetic field applied to the capsule endoscope 2 in the subject 1 by the magnetic field generator 15 on the basis of the monitoring result of the change over time of the current value. The control unit 14 obtains the time rate of change of the magnetic field detected by the magnetic field change detector 17 as the change of the magnetic field formed by the magnetic field generator 15 to guide the capsule endoscope 2 inside the subject 1.

Next, the control unit 14 determines whether or not the change of the magnetic field applied to the subject 1 (specifically, the capsule endoscope 2 inside an organ) by the magnetic field generator 15 is smaller than or equal to a predetermined threshold value on the basis of the detection result of the change of the magnetic field obtained from the magnetic field change detector 17 (step S102). Specifically, the control unit 14 compares the time rate of change of the magnetic field obtained from the magnetic field change detector 17 in step S101 with a predetermined threshold value set in advance, and determines whether or not the time rate of change of the magnetic field is smaller than or equal to the threshold value.

Here, when the strength of the magnetic field applied to the capsule endoscope 2 inside the subject 1 by the magnetic field generator 15 increases rapidly over time, an eddy current due to the magnetic field is generated on the body surface of the subject 1. Therefore, when the control unit 14 determines that the time rate of change of the magnetic field is not smaller than or equal to the threshold value (exceeds the threshold value) (step S102, No), the control unit 14 recognizes that an eddy current due to the magnetic field is generated on the body surface of the subject 1. In the timing when the eddy current is generated, the control unit 14 controls the position calculator 9 and the image processing unit 7 to stop the position calculation processing and the image generation processing described above (step S103). Thereafter, the process returns to step S101 described above, and the control unit 14 repeats the processing procedure of step S101 and the following steps.

The control unit 14 eliminates the noise-containing signal (in other words, an electrical signal including a signal component of eddy current) from an electrical signal to be processed by the position calculation processing or the image generation processing described above by performing control to stop both of the position calculation processing of the position calculator 9 and the image generation processing of the image processing unit 7 in the timing when the eddy current is generated on the body surface of the subject 1.

On the other hand, when the control unit 14 determines that the time rate of change of the magnetic field obtained from the magnetic field change detector 17 in step S101 is smaller than or equal to the threshold value (step S102, Yes), the control unit 14 recognizes that the eddy current is not generated on the body surface of the subject 1. In the timing when the eddy current is not generated, the control unit 14 controls the position calculator 9 and the image processing unit 7 to perform the position calculation processing and the image generation processing described above (step S104). Thereafter, the process returns to step S101 described above, and the control unit 14 repeats the processing procedure of step S101 and the following steps.

Next, operations of the magnetic field change detector 17 and the control unit 14 when eliminating the signal components of the eddy current due to the magnetic field will be specifically described. FIG. 5 is a diagram illustrating the change over time of the strength of the magnetic field applied from the magnetic field generator 15 to the capsule endoscope 2 inside the subject 1. Hereinafter, the operations of the magnetic field change detector 17 and the control unit 14 when eliminating the signal components of the eddy current due to the magnetic field will be specifically described with reference to the above-described FIG. 1 and FIG. 5.

When a magnetic field strength H applied to the capsule endoscope 2 inside the subject 1 by the magnetic field generator 15 changes over time t as shown in FIG. 5, the magnetic field change detector 17 detects the time rate of change of the magnetic field strength H with respect to the time t by monitoring the value of the current supplied from the signal generator 16 to the magnetic field generator 15 as described above. The magnetic field change detector 17 transmits the time rate of change of the magnetic field strength H to the control unit 14 as the detection result of the change of the magnetic field. The control unit 14 obtains the time rate of change of the magnetic field strength H detected by the magnetic field change detector 17, and controls the image generation processing timing of the image processing unit 7 and the position calculation processing timing of the position calculator 9 described above on the basis of the obtained time rate of change of the magnetic field strength H.

Here, as illustrated by the change of the magnetic field strength H in a period between t1 and t2 or a period between t3 and t4 shown in FIG. 5, when the magnetic field strength H applied to the capsule endoscope 2 inside the subject 1 changes rapidly over the time t, an eddy current is generated in the body or on the body surface of the subject 1. In this case, a voltage caused by the electrical signal from the capsule endoscope 2 that performs the human body communication and a voltage caused by the eddy current are induced in each of the plurality of electrode pads 5 disposed on the body surface of the subject 1. Therefore, the receiving unit 6 receives the noise-containing signal via the plurality of electrode pads 5 in the period between t1 and t2 or the period between t3 and t4. As a result, it is difficult for the electric potential distribution acquisition unit 8 to obtain correct electric potential distribution on the subject 1 (specifically, electric potential distribution caused by the electrical signal from the capsule endoscope 2) due to the signal components of the eddy current included in the noise-containing signal. Because of this, it is difficult for the position calculator 9 to correctly calculate the position of the capsule endoscope 2 inside the subject 1. Also, the image processing unit 7 is prevented from performing the image generation processing for generating (reconstructing) an in-vivo image of the subject 1 due to the signal components of the eddy current.

On the other hand, in an eddy current generation period as illustrated by the period between t1 and t2 or the period between t3 and t4, the control unit 14 eliminates the noise-containing signal from the electrical signal to be processed by stopping both the image generation processing of the image processing unit 7 and the position calculation processing of the position calculator 9 described above. Specifically, for example, when the control unit 14 obtains the time rate of change of the magnetic field strength H from the magnetic field change detector 17 in the period between t1 and t2 or the period between t3 and t4, the control unit 14 determines that the obtained time rate of change of the magnetic field strength H exceeds a predetermined threshold value. In this case, the control unit 14 recognizes that an eddy current due to the magnetic field is generated on the body surface of the subject 1 in the period between t1 and t2 or the period between t3 and t4. On the other hand, in the timing when the eddy current due to the magnetic field is generated (that is, the period between t1 and t2 or the period between t3 and t4), the control unit 14 stops both the image generation processing of the image processing unit 7 and the position calculation processing of the position calculator 9, and thereby eliminates the signal components of the eddy current from the electrical signal to be processed by the image generation processing or the position calculation processing (that is, the electrical signals detected by the plurality of electrode pads 5). As a result, the control unit 14 prevents the image generation processing and the position calculation processing from being disturbed by the signal components of the eddy current.

As described above, the first embodiment of the invention is configured so that the time rate of change of the magnetic field formed to guide the capsule endoscope inserted into an organ of the subject is detected, when the time rate of change of the magnetic field is smaller than or equal to a predetermined threshold value, the image generation processing for generating (reconstructing) an in-vivo image received from the capsule endoscope via the electrode pads on the body surface of the subject and the position calculation processing for calculating the position of the capsule endoscope inside the subject 1 are performed, and when the time rate of change of the magnetic field exceeds the predetermined threshold value, the image generation processing and the position calculation processing are stopped. Therefore, in the period when an eddy current is generated on the body surface of the subject by the change of the magnetic field applied to the capsule endoscope inside the subject, the image generation processing and the position calculation processing are reliably stopped, and thereby an electrical signal (noise-containing signal) including the signal components of the eddy current as a noise component is prevented from being used in the image generation processing and the position calculation processing, so that the noise-containing signal can be eliminated. As a result, when magnetically guiding the capsule endoscope inside the subject, the signal components of the eddy current due to the magnetic field can be eliminated from the electrical signals detected by the electrode pads on the body surface, so that it is possible to realize a capsule guidance system in which the position of the capsule endoscope inside the subject can be detected and in-vivo images of the subject can be obtained without being disturbed by the eddy current.

### Second Embodiment

Next, a second embodiment of the present invention will be described. While, in the first embodiment described above, the position calculation processing of the position calculator 9 is stopped so as to eliminate the signal components of the eddy current due to the magnetic field, in the second embodiment, when the eddy current due to the magnetic field is generated on the body surface of the subject 1, the signal components of the eddy current are eliminated by subtracting the signal component of the eddy current from each voltage detected by the plurality of electrode pads 5.

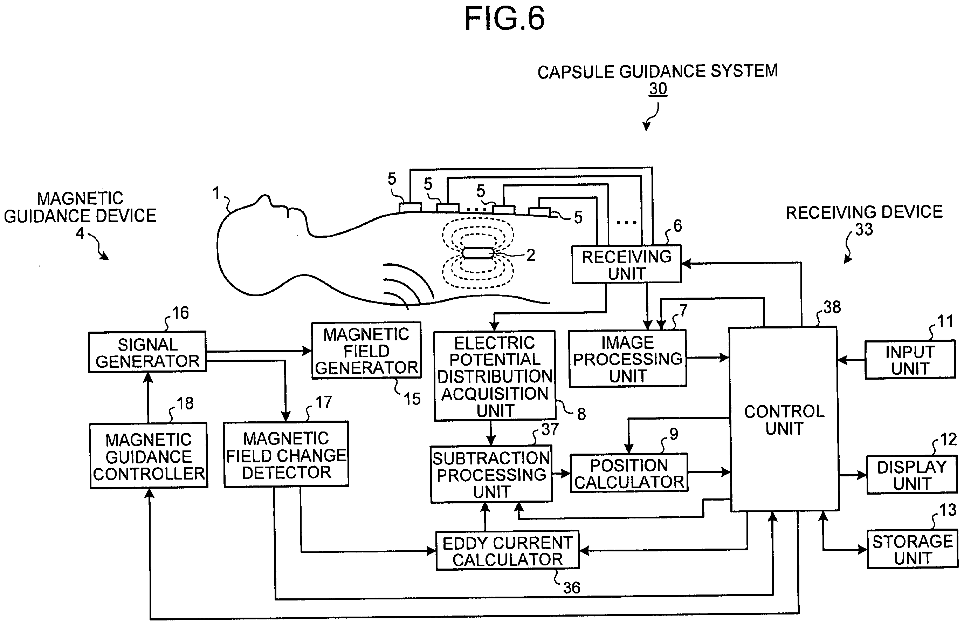

FIG. 6 is a block diagram schematically showing a configuration example of a capsule guidance system according to the second embodiment of the present invention. As shown in FIG. 6, a capsule guidance system 30 according to the second embodiment includes a receiving device 33 instead of the receiving device 3 in the capsule guidance system 10 according to the first embodiment described above. The receiving device 33 includes a control unit 38 instead of the control unit 14 in the receiving device 3 of the first embodiment described above, and further includes an eddy current calculator 36 for calculating the signal components of the eddy current due to the magnetic field formed in the subject 1 and a subtraction processing unit 37 for subtracting the signal components of the eddy current from the voltages of each electrical signal detected by the plurality of electrode pads 5. In this case, the electric potential distribution acquisition unit 8 and the position calculator 9 described above are connected to each other via the subtraction processing unit 37. The other configuration is the same as that of the first embodiment, and the same constituent elements are given the same reference numerals.

When an eddy current is generated in the subject 1, the eddy current calculator 36 calculates the signal components of the eddy current. Specifically, the eddy current calculator 36 is connected to the magnetic field change detector 17, and obtains the time rate of change of the magnetic field applied from the magnetic field generator 15 to the capsule endoscope 2 inside the subject 1 from the magnetic field change detector 17. The eddy current calculator 36 previously knows body information such as the size of the body of the subject 1 and position information of each electrode pad 5 on the body surface of the subject 1. The eddy current calculator 36 calculates the eddy current generated in the body or on the body surface of the subject 1 by the change of the magnetic field on the basis of the known body information of the subject 1 and position information of each electrode pad 5, and the time rate of change of the magnetic field obtained from the magnetic field change detector 17. In this case, the eddy current calculator 36 calculates eddy currents generated near the positions of each of the plurality of electrode pads 5 dispersed and disposed on the body surface of the subject 1. The eddy current calculator 36 calculates the signal components of the eddy current detected by each of the plurality of electrode pads 5 as error voltages due to the eddy current on the basis of the calculation result of the eddy current. The error voltages due to the eddy current are error voltages related to the electric potential distribution on the subject 1. The eddy current calculator 36 transmits the calculation result of the signal components of the eddy current to the subtraction processing unit 37. The calculation processing timing of the eddy current calculator 36 is controlled by the control unit 38.

When an eddy current is generated in the subject 1, the subtraction processing unit 37 subtracts the error voltages due to the eddy current from the electric potential distribution on the subject 1 acquired by the electric potential distribution acquisition unit 8. Specifically, the subtraction processing unit 37 is connected to the electric potential distribution acquisition unit 8, and receives the electric potential distribution on the subject 1 from the electric potential distribution acquisition unit 8. Also, the subtraction processing unit 37 receives the error voltages due to the eddy current (the signal components of the eddy current) calculated by the eddy current calculator 36. Here, the electric potential distribution on the subject 1 acquired by the electric potential distribution acquisition unit 8 when an eddy current is generated in the subject 1 includes signal components of the eddy current (that is, error voltages due to the eddy current). The subtraction processing unit 37 subtracts the error voltages due to the eddy current calculated by the eddy current calculator 36 from each voltage forming the electric potential distribution on the subject 1 (that is, each voltage detected by the plurality of electrode pads 5). The subtraction processing unit 37 calculates correct electric potential distribution on the subject 1 that does not include the error voltages due to the eddy current (that is, electric potential distribution formed on the body surface of the subject 1 by the human body communication of the capsule endoscope 2) by the subtraction processing. In this case, the subtraction processing unit 37 performs subtraction processing for subtracting the signal components of the eddy current from the voltages of each electrical signal detected by the plurality of electrode pads 5, and thereby calculates the voltages of the electrical signals from the capsule endoscope 2 detected by the plurality of electrode pads 5. The subtraction processing unit 37 is connected to the position calculator 9, and transmits the result of the subtraction processing to the position calculator 9. The subtraction processing timing of the subtraction processing unit 37 is controlled by the control unit 38.

The control unit 38 controls the calculation processing timing of the eddy current calculator 36 and the subtraction processing timing of the subtraction processing unit 37 so that the position of the capsule endoscope 2 inside the subject 1 can be calculated without being affected by the eddy current generated on the body surface of the subject 1. Specifically, when an eddy current is generated on the body surface of the subject 1, the control unit 38 causes the eddy current calculator 36 to perform the calculation processing for calculating the error voltages due to the eddy current (the signal components of the eddy current) instead of stopping the position calculation processing of the position calculator 9 as described in the first embodiment, and causes the subtraction processing unit 37 to perform the subtraction processing for subtracting the error voltages due to the eddy current from the electric potential distribution on the subject 1. On the other hand, when an eddy current is not generated on the body surface of the subject 1, the control unit 38 stops the calculation processing of the eddy current calculator 36 and the subtraction processing of the subtraction processing unit 37. In this case, the control unit 38 controls the subtraction processing unit 37 to transmit the electric potential distribution on the subject 1 acquired by the electric potential distribution acquisition unit 8 to the position calculator 9. The other functions of the control unit 38 are the same as those of the control unit 14 of the first embodiment described above.

Although, when an eddy current is generated on the body surface of the subject 1, the control unit 38 causes the eddy current calculator 36 to perform the calculation processing and causes the subtraction processing unit 37 to perform the subtraction processing, it is not limited to this. The control unit 38 may cause the eddy current calculator 36 to perform the calculation processing every time the eddy current calculator 36 obtains the time rate of change of the magnetic field from the magnetic field change detector 17, the subtraction processing unit 37 to perform the subtraction processing only when the time rate of change of the magnetic field detected by the magnetic field change detector 17 exceeds a predetermined threshold value. In other words, the control unit 38 only has to control at least the subtraction processing timing of the subtraction processing unit 37 on the basis of the time rate of change of the magnetic field detected by the magnetic field change detector 17.

The position calculator 9 controlled by the control unit 38 calculates the position of the capsule endoscope 2 inside the subject 1 on the basis of the electric potential distribution on the subject 1 every time the position calculator 9 receives the electric potential distribution on the subject 1 from the subtraction processing unit 37 regardless whether or not an eddy current is generated on the body surface of the subject 1. Specifically, when an eddy current is generated on the body surface of the subject 1, the position calculator 9 calculates the position of the capsule endoscope 2 on the basis of the electric potential distribution on the subject 1 from which the error voltages due to the eddy current are eliminated by the subtraction processing unit 37, and when an eddy current is not generated on the body surface of the subject 1, the position calculator 9 calculates the position of the capsule endoscope 2 on the basis of the electric potential distribution transmitted from the electric potential distribution acquisition unit 8 by the subtraction processing unit 37. In either case, the position calculator 9 can calculate the position of the capsule endoscope 2 on the basis of the electric potential distribution formed on the body surface of the subject 1 by the potential signal from the capsule endoscope 2.

Here, the above-described magnetic field change detector 17, the eddy current calculator 36, the subtraction processing unit 37, and the control unit 38 form an eliminating means for eliminating the signal components of the eddy current from the electrical signals detected by the plurality of electrode pads 5. Specifically, the magnetic field change detector 17 detects the time rate of change of the magnetic field applied to the capsule endoscope 2 inside the subject 1, and transmits the detected time rate of change of the magnetic field to the eddy current calculator 36 and the control unit 38. The control unit 38 controls the image generation processing timing of the image processing unit 7, the calculation processing timing of the eddy current calculator 36, and the subtraction processing timing of the subtraction processing unit 37 on the basis of the time rate of change of the magnetic field transmitted from the magnetic field change detector 17. In this case, in a state in which an eddy current is generated in the subject 1 due to a rapid change of the strength of the magnetic field applied to the capsule endoscope 2, the control unit 38 stops the image generation processing of the image processing unit 7, causes the eddy current calculator 36 to perform the calculation processing for calculating the error voltages due to the eddy current, and causes the subtraction processing unit 37 to perform the subtraction processing for subtracting the error voltages due to the eddy current from the electric potential distribution on the subject 1. As a result, the control unit 38 can eliminate the signal component of the eddy current from the electrical signal transmitted from the receiving unit 6 to the image processing unit 7, and also can eliminate the signal components of the eddy current (that is, the error voltages due to the eddy current) from the electric potential distribution on the subject 1 to be transmitted to the position calculator 9.

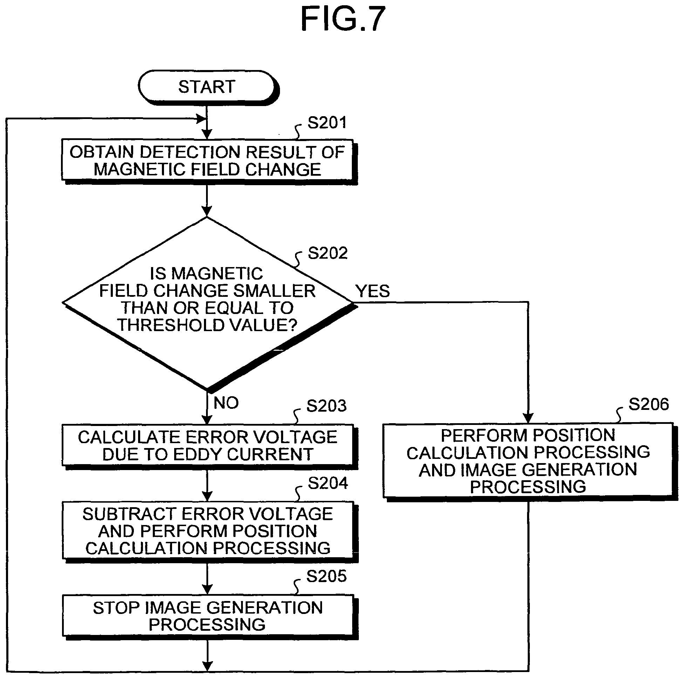

Next, an operation of the control unit 38 which forms an eliminating means for eliminating the signal components of the eddy current will be described. FIG. 7 is a flowchart illustrating a processing procedure of the control unit 38 to eliminate the signal components of the eddy current.

As shown in FIG. 7, in the same manner as in steps S101 and S102 described above (see FIG. 4), the control unit 38 receives the detection result of the magnetic field change, which is the time rate of change of the magnetic field, detected by the magnetic field change detector 17 (step S201), and determines whether or not the obtained time rate of change of the magnetic field is smaller than or equal to a predetermined threshold value (step S202). In this case, the eddy current calculator 36 obtains the same time rate of change of the magnetic field as that obtained by the control unit 38 from the magnetic field change detector 17.

Here, when the strength of the magnetic field applied to the capsule endoscope 2 inside the subject 1 by the magnetic field generator 15 increases rapidly over time, an eddy current due to the magnetic field is generated on the body surface of the subject 1. That is, when the control unit 38 determines that the time rate of change of the magnetic field is not smaller than or equal to the threshold value (exceeds the threshold value) (step S202, No), the control unit 38 recognizes that an eddy current due to the magnetic field is generated on the body surface of the subject 1.

In the timing when the eddy current is generated, the control unit 38 causes the eddy current calculator 36 to perform the calculation processing for calculating the signal components of the eddy current, that is, the error voltages due to the eddy current (step S203), subtracts the calculated error voltages due to the eddy current, and performs the position calculation processing (step S204). In step S203, under the control of the control unit 38, the eddy current calculator 36 calculates the error voltages due to the eddy current on the basis of the time change rate of the magnetic field obtained from the magnetic field change detector 17, and transmits the calculated error voltages due to the eddy current to the subtraction processing unit 37. In step S204, the control unit 38 causes the subtraction processing unit 37 to perform the subtraction processing for subtracting the error voltages due to the eddy current calculated by the eddy current calculator 36 from the electric potential distribution on the subject 1, and causes the position calculator 9 to perform the position calculation processing for calculating the position of the capsule endoscope 2 on the basis of the electric potential distribution on the subject 1 from which the error voltages due to the eddy current are eliminated by the subtraction processing.

Next, the control unit 38 controls the image processing unit 7 to stop the above-described image generation processing (step S205). Thereafter, the process returns to step S201 described above, and the control unit 38 repeats the processing procedure of step S201 and the following steps. The control unit 38 may perform the control to stop the image generation processing of the image processing unit 7 before performing the calculation processing of the eddy current calculator 36, before performing the subtraction processing of the subtraction processing unit 37, or before performing the position calculation processing of the position calculator 9.

On the other hand, when the control unit 38 determines that the time rate of change of the magnetic field obtained from the magnetic field change detector 17 in step S201 is smaller than or equal to the threshold value (step S202, Yes), the control unit 38 recognizes that the eddy current is not generated on the body surface of the subject 1. In the timing when the eddy current is not generated, the control unit 38 controls the position calculator 9 and the image processing unit 7 to perform the position calculation processing and the image generation processing described above (step S206). In this case, the control unit 38 stops the calculation processing of the eddy current calculator 36 and the subtraction processing of the subtraction processing unit 37, and controls the subtraction processing unit 37 to transmit the electric potential distribution on the subject 1 acquired by the electric potential distribution acquisition unit 8 to the position calculator 9. Thereafter, the process returns to step S201 described above, and the control unit 38 repeats the processing procedure of step S201 and the following steps.

Next, the operations of the eliminating means (that is, the magnetic field change detector 17, the eddy current calculator 36, the subtraction processing unit 37, and the control unit 38) when eliminating the signal components of the eddy current due to the magnetic field will be specifically described with reference to the above-described FIG. 5 and FIG. 6. In the same manner as in the first embodiment described above, the control unit 38 controls the image generation processing timing of the image processing unit 7 on the basis of the time rate of change of the magnetic field strength H, and thereby prevents the position calculation processing from being disturbed by the signal components of the eddy current. Therefore, the description of the operation of the control unit 38 when controlling the image generation processing timing will be omitted.

When the magnetic field strength H applied to the capsule endoscope 2 inside the subject 1 by the magnetic field generator 15 changes over time t as shown in FIG. 5, as described above, the magnetic field change detector 17 detects the time rate of change of the magnetic field strength H with respect to the time t. The magnetic field change detector 17 transmits the time rate of change of the magnetic field strength H to the eddy current calculator 36 and the control unit 38 as the detection result of the change of the magnetic field. The control unit 38 obtains the time rate of change of the magnetic field strength H detected by the magnetic field change detector 17, and controls the calculation processing timing of the eddy current calculator 36 and the subtraction processing timing of the subtraction processing unit 37 described above on the basis of the obtained time rate of change of the magnetic field strength H.

Here, as illustrated by the change of the magnetic field strength H in the period between t1 and t2 or the period between t3 and t4 shown in FIG. 5, when the magnetic field strength H applied to the capsule endoscope 2 inside the subject 1 changes rapidly over the time t, an eddy current is generated in the body or on the body surface of the subject 1. In this case, it is difficult for the electric potential distribution acquisition unit 8 to acquire correct electric potential distribution on the subject 1 formed by the electrical signal from the capsule endoscope 2, and the electric potential distribution acquisition unit 8 acquires electric potential distribution on the subject 1 including the error voltages due to the eddy current.

On the other hand, in an eddy current generation period as illustrated by the period between t1 and t2 or the period between t3 and t4, the control unit 38 eliminates the error voltages due to the eddy current from the electric potential distribution on the subject 1 which is used for the position calculation processing of the position calculator 9 by causing the eddy current calculator 36 to perform the calculation processing and the subtraction processing unit 37 to perform the subtraction processing.

Specifically, for example, when the control unit 38 obtains the time rate of change of the magnetic field strength H from the magnetic field change detector 17 in the period between t1 and t2 or the period between t3 and t4, the control unit 38 determines that the obtained time rate of change of the magnetic field strength H exceeds a predetermined threshold value. In this case, the control unit 38 recognizes that an eddy current due to the magnetic field is generated on the body surface of the subject 1 in the period between t1 and t2 or the period between t3 and t4. In the timing when the eddy current due to the magnetic field is generated (that is, the period between t1 and t2 or the period between t3 and t4), the control unit 38 causes the eddy current calculator 36 to perform the calculation processing and the subtraction processing unit 37 to perform the subtraction processing. On the basis of the control of the control unit 38, the eddy current calculator 36 calculates the error voltages due to the eddy current in the period between t1 and t2 or the period between t3 and t4, and transmits the calculated error voltages due to the eddy current to the subtraction processing unit 37. The subtraction processing unit 37 subtracts the error voltages due to the eddy current calculated by the eddy current calculator 36 from the electric potential distribution on the subject 1 to calculate the correct electric potential distribution on the subject 1 from which the error voltages due to the eddy current are eliminated. The control unit 38 controls the position calculator 9 to calculate the position of the capsule endoscope 2 on the basis of the correct electric potential distribution on the subject 1 from which the error voltages due to the eddy current are eliminated by the subtraction processing unit 37. In this way, the control unit 38 eliminates the signal components of the eddy current from the electrical signal to be processed in the position calculation processing. As a result, the control unit 38 prevents the position calculation processing from being disturbed by the signal components of the eddy current.

As described above, the second embodiment of the invention includes the eddy current calculator for calculating the signal components of the eddy current generated in the subject as the error voltages due to the eddy current and the subtraction processing unit for subtracting the error voltages due to the eddy current calculated by the eddy current calculator from the electric potential distribution on the subject, and in the second embodiment of the present invention, when the time rate of change of the magnetic field formed to guide the capsule endoscope inserted into an organ of the subject exceeds a predetermined threshold value, the calculation processing of the eddy current calculator and the subtraction processing of the subtraction processing unit are performed, and the position calculation processing for calculating the position of the capsule endoscope is performed on the basis of the electric potential distribution on the subject from which the error voltages due to the eddy current are eliminated. The other configuration of the second embodiment is configured in approximately the same manner as in the first embodiment described above. Therefore, in addition to the same operational effects as those of the first embodiment described above, even in a period when an eddy current is generated on the body surface of the subject by the change of the magnetic field applied to the capsule endoscope inside the subject, it is possible to calculate the position of the capsule endoscope on the basis of the correct electric potential distribution on the subject from which the error voltages due to the eddy current are eliminated. As a result, while the same operational effects as those of the first embodiment described above are obtained, it is possible to continuously detect the position of the capsule endoscope inside the subject regardless of whether or not an eddy current is generated in the subject, and the capsule endoscope can be easily guided to a desired position inside the subject on the basis of the position detection result.

### Third Embodiment

Next, a third embodiment of the present invention will be described. While, in the first embodiment described above, the signal components of the eddy current are eliminated by stopping the image generation processing of the image processing unit 7 and the position calculation processing of the position calculator 9 when the eddy current is generated in the subject 1, in the third embodiment, the signal components of the eddy current are eliminated by a filter from each electrical signal detected by the plurality of electrode pads 5.

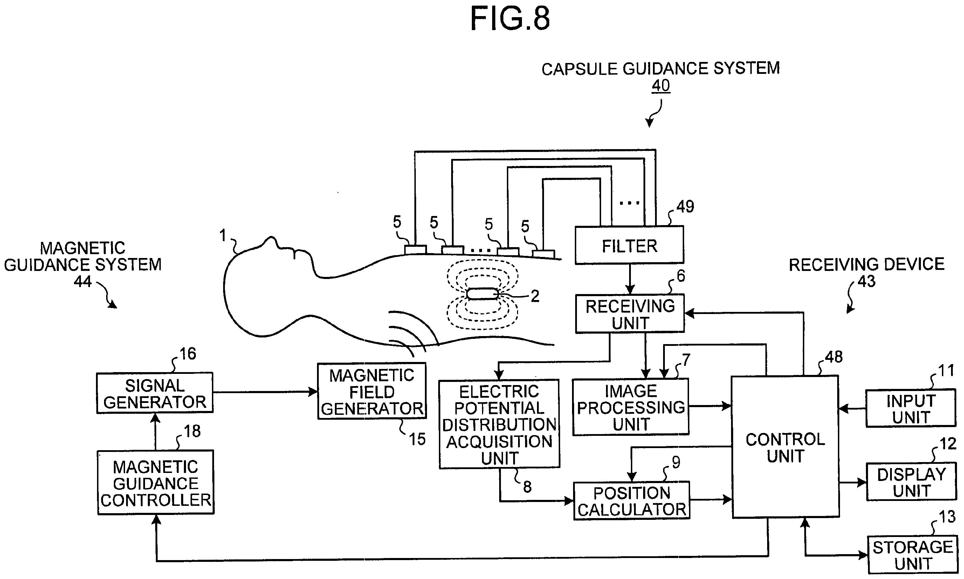

FIG. 8 is a block diagram schematically showing a configuration example of a capsule guidance system according to the third embodiment of the present invention. As shown in FIG. 8, a capsule guidance system 40 according to the third embodiment includes a receiving device 43 instead of the receiving device 3 in the capsule guidance system 10 according to the first embodiment described above and a magnetic guidance device 44 instead of the magnetic guidance device 4. The receiving device 43 does not eliminate the signal components of the eddy current by controlling operation timings of the image processing unit 7 and the position calculator 9 on the basis of the time rate of change of the magnetic field, but eliminates the signal components of the eddy current by a filter. Specifically, the receiving device 43 includes a control unit 48 instead of the control unit 14 in the receiving device 3 of the first embodiment described above, and further includes a filter 49 for eliminating the signal component of the eddy current from each electrical signal detected by the plurality of electrode pads 5. On the other hand, the magnetic guidance device 44 has approximately the same configuration as that in which the magnetic field change detector 17 is removed from the magnetic guidance device 4. The other configuration is the same as that of the first embodiment, and the same constituent elements are given the same reference numerals.

The filter 49 functions as an eliminating means for eliminating the signal components of the eddy current from the electrical signals detected by the plurality of electrode pads 5. Specifically, the filter 49 is an analogue filter such as a band-pass filter or a high-pass filter. The filter 49 eliminates signal components of the frequency band of the eddy current (that is, the signal components of the eddy current) generated in the subject 1 by the change of the magnetic field applied from the magnetic field generator 15 to the capsule endoscope 2 inside the subject 1. The filter 49 is connected to the plurality of electrode pads 5, and eliminates the signal component of the eddy current from each electrical signal detected by the plurality of electrode pads 5. Also, the filter 49 is connected to the receiving unit 6 and transmits electrical signals from which the signal components of the eddy current are eliminated, that is, the electrical signals from the capsule endoscope 2 detected by the plurality of electrode pads 5, to the receiving unit 6.

The control unit 48 does not have a function to control operation timings of the image processing unit 7 and the position calculator 9 on the basis of the time rate of change of the magnetic field described above. Instead of such a control function, every time the receiving unit 6 demodulates an image signal including an in-vivo image of the subject 1, the control unit 48 controls the image processing unit 7 to perform image generation processing for generating (reconstructing) the in-vivo image of the subject 1 on the basis of the image signal. Every time the receiving unit 6 receives the electrical signals detected by the plurality of electrode pads 5, in other words, every time the electric potential distribution acquisition unit 8 acquires the electric potential distribution on the subject 1, the control unit 48 controls the position calculator 9 to perform the position calculation processing for calculating the position of the capsule endoscope 2 on the basis of the electric potential distribution on the subject 1. The other functions of the control unit 48 are the same as those of the control unit 14 of the first embodiment described above.

Here, as described above, the eddy current in the body or on the body surface of the subject 1 is generated by the change of the magnetic field applied to the capsule endoscope 2 inside the subject 1 by the magnetic field generator 15. Therefore, the signal components of the eddy current are detected by the plurality of electrode pads 5 as AC signals having the same frequency band as that of the magnetic field (for example, 100 Hz or less). On the other hand, the electrical signal from the capsule endoscope 2 that performs the human body communication is a signal having a frequency band (for example, about 1 MHz to 10 MHz) sufficiently higher than the frequency band of the signal components of the eddy current. Therefore, the filter 49 having a function to eliminate signal components having a frequency band lower than the frequency band of the electrical signal from the capsule endoscope 2 can easily eliminate the signal components of the eddy current from the electrical signals detected by the plurality of electrode pads 5. As a result, the filter 49 can prevent the image generation processing of the image processing unit 7 and the position calculation processing of the position calculator 9 from being disturbed by the signal components of the eddy current.

As described above, in the third embodiment of the invention, the filter that eliminates signal components having a frequency band lower than the frequency band of the electrical signals from the capsule endoscope performing the human body communication and the plurality of electrode pads disposed on the body surface of the subject are connected to each other, and the electrical signals detected by the plurality of electrode pads are received through the filter. Therefore, the signal components of the eddy current, which are noise components having a frequency band lower than that of the electric signals from the capsule endoscope, can be eliminated by the filter. As a result, it is possible to realize a capsule guidance system with a simple configuration in which, when magnetically guiding the capsule endoscope inside the subject, the position of the capsule endoscope inside the subject can be detected and in-vivo images of the subject can be obtained without being disturbed by the eddy current generated in the subject.

Since the position of the capsule endoscope is calculated on the basis of the electrical signals received through the filter, it is possible to continuously detect the position of the capsule endoscope inside the subject regardless of whether or not an eddy current is generated in the subject, and the capsule endoscope can be easily guided to a desired position inside the subject on the basis of the position detection result.

Further, since an in-vivo image of the subject is generated on the basis of an image signal demodulated from the electrical signal received through the filter, it is possible to continuously obtain in-vivo images inside the subject regardless of whether or not an eddy current is generated in the subject. As a result, a greater amount of image data for observing the inside of organs of the subject can be obtained, so that it is possible to prevent a situation from occurring in which an in-vivo image of a portion of interest such as a bleeding site or a lesion site is missed.

### Fourth Embodiment

Next, a fourth embodiment of the present invention will be described. While, in the third embodiment described above, the signal components of the eddy current are eliminated from each electrical signal detected by the plurality of electrode pads 5 by an analog filter, in the fourth embodiment, the signal components of the eddy current are eliminated by a digital filter that performs FFT processing on each electrical signal detected by the plurality of electrode pads 5.

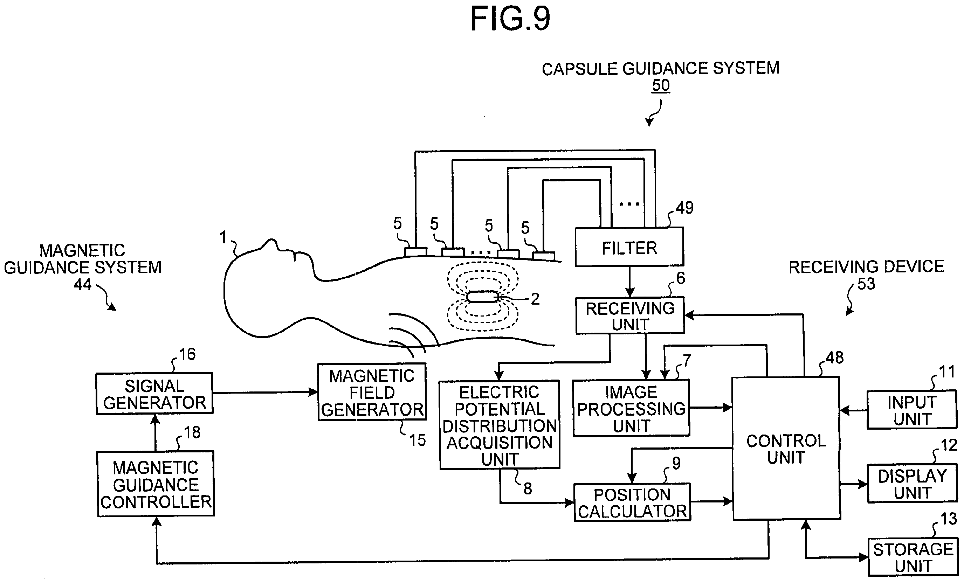

FIG. 9 is a block diagram schematically showing a configuration example of a capsule guidance system according to the fourth embodiment of the present invention. As shown in FIG. 9, a capsule guidance system 50 according to the fourth embodiment includes a receiving device 53 instead of the receiving device 43 in the capsule guidance system 40 according to the third embodiment described above. The receiving device 53 includes a filter 54 for limiting a frequency band instead of the filter 49 in the receiving device 43 of the third embodiment described above, and further includes a receiving unit 55 including a digital filter instead of the receiving unit 6. The other configuration is the same as that of the third embodiment, and the same constituent elements are given the same reference numerals.

The filter 54 performs band limitation of the electrical signals received by the receiving unit 55 via the plurality of electrode pads 5. Specifically, the filter 54 is, for example, a band-pass filter, and electrically connected to the plurality of electrode pads 5. The filter 54 eliminates signal components other than signal components having a predetermined frequency band in the electrical signals detected by the plurality of electrode pads 5 (specifically, electrical signals including at least the electrical signal from the capsule endoscope 2). The filter 54 is electrically connected to the receiving unit 55, and transmits electrical signals within a predetermined frequency band extracted by eliminating the signal components other than the signal components having the predetermined frequency band to the receiving unit 55. In this way, the filter 54 limits the frequency band of electrical signals on which analog-digital conversion processing (A/D conversion processing) is performed by an A/D converter 55a of the receiving unit 55 described below.

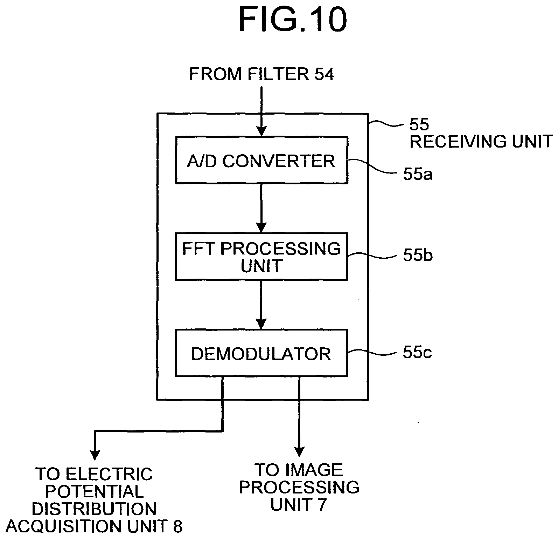

The receiving unit 55 includes a digital filter processing function for eliminating the signal components of the eddy current by performing digital processing such as the FFT processing, a demodulation processing function for demodulating an image signal on the basis of an electrical signal from which the signal component of the eddy current is eliminated (that is, the electrical signal from the capsule endoscope 2), and a voltage transmission processing function for transmitting voltages obtained by eliminating the signal components of the eddy current from each voltage detected by the plurality of electrode pads 5 (that is, voltages induced in each electrode pad 5 by the electrical signal from the capsule endoscope 2) to the electric potential distribution acquisition unit 8. For example, as shown in FIG. 10, the receiving unit 55 includes the A/D converter 55a, an FFT processing unit 55b, and a demodulator 55c.

The A/D converter 55a converts each electrical signal (analog signal) received from the plurality of electrode pads 5 through the filter 54 into a digital signal. Specifically, the A/D converter 55a converts an analog signal whose band is limited by the above-described filter 54 into a digital signal and transmits the converted digital signal to the FFT processing unit 55b. In summary, the A/D converter 55a transmits a digital signal having a frequency band which is limited by the above-described filter 54 to the FFT processing unit 55b.

The FFT processing unit 55b functions as a digital filter for converting each electrical signal detected by the plurality of electrode pads 5 into frequency components and eliminating a frequency component of the eddy current from the converted frequency components. Specifically, the FFT processing unit 55b performs FFT processing on a digital signal A/D-converted by the A/D converter 55a to convert the digital signal from time components into frequency components, and divides the converted frequency components into a frequency component corresponding to the electrical signal from the capsule endoscope 2 (frequency component of a human body communication signal) and a frequency component corresponding to the signal component of the eddy current (frequency component of the eddy current). The FFT processing unit 55b extracts the frequency component of the human body communication signal by eliminating the frequency component of the eddy current from the frequency components divided by the FFT processing. Thereafter, the FFT processing unit 55b converts the frequency component of the human body communication signal into a time component by performing inverse FFT processing on the frequency component of the human body communication signal extracted in the manner described above. In this way, the FFT processing unit 55b extracts the electrical signals from the capsule endoscope 2 (specifically, the electrical signals from the capsule endoscope 2 detected by each of the plurality of electrode pads 5) from the digital signal A/D-converted by the A/D converter 55a described above. The FFT processing unit 55b transmits the electrical signals from the capsule endoscope 2 extracted in the manner described above to the demodulator 55c.

The demodulator 55c includes a demodulation processing function for demodulating an image signal that includes an in-vivo image of the subject 1 captured by the capsule endoscope 2, and a voltage transmission processing function for transmitting voltage values induced in the electrode pairs of the plurality of electrode pads 5 by the electrical signal from the capsule endoscope 2 to the electric potential distribution acquisition unit 8. Specifically, the demodulator 55c obtains the electrical signals from which the signal components of the eddy current are eliminated by the FFT processing unit 55b (that is, the electrical signals from the capsule endoscope 2 detected by each of the plurality of electrode pads 5). The demodulator 55c selects an electrical signal corresponding to a highest voltage among the voltages detected by the plurality of electrode pads 5 from the obtained electrical signals from the capsule endoscope 2, and performs demodulation processing or the like on the selected electrical signal to demodulate the electrical signal into an image signal. The demodulator 55c transmits the demodulated image signal (that is, the image signal including an in-vivo image of the subject 1) to the image processing unit 7. On the other hand, the demodulator 55c transmits (notifies of) voltage values of the electrical signals obtained from the above-described FFT processing unit 55b (specifically, voltage values of the electrical signals from the capsule endoscope 2 detected by each of the plurality of electrode pads 5) to the electric potential distribution acquisition unit 8.

Here, the above-described filter 54 and the FFT processing unit 55b form an eliminating means for eliminating the signal components of the eddy current from the electrical signals detected by the plurality of electrode pads 5. Specifically, the filter 54 limits the frequency band of the electrical signals detected by the plurality of electrode pads 5, and the FFT processing unit 55b converts the electrical signals whose frequency band is limited by the filter 54 into frequency components and eliminates the frequency component of the eddy current from the converted frequency components to extract the frequency component of the human body communication signal. The frequency components converted by the FFT processing unit 55b include the frequency component of the human body communication signal and the frequency component of the eddy current which is a frequency component lower than the frequency component of the human body communication signal. Therefore, even when the frequency component of the human body communication signal has a frequency band near the frequency band of the frequency component of the eddy current, the FFT processing unit 55b can divide the electrical signals, whose frequency band is limited by the filter 54, into the frequency component of the human body communication signal and the frequency component of the eddy current by performing the FFT processing on the electrical signals whose frequency band is limited. The FFT processing unit 55b can reliably eliminate the frequency component of the eddy current by eliminating frequency components lower than the frequency component of the human body communication signal from the frequency components divided by the FFT processing. As a result, the FFT processing unit 55b can prevent the image generation processing of the image processing unit 7 and the position calculation processing of the position calculator 9 from being disturbed by the signal components of the eddy current described above.

As described above, in the fourth embodiment of the invention, the frequency band of the electrical signals detected by the plurality of electrode pads disposed on the body surface of the subject is limited by a band-pass filter, the electrical signals whose frequency band is limited are divided into the frequency component of the human body communication signal and the frequency component of the eddy current by a digital filter having a frequency analysis function such as the FFT processing, and the frequency component of the eddy current which is a signal component having a frequency band lower than the frequency component of the human body communication signal is eliminated. Therefore, even when the frequency component of the human body communication signal has a frequency band near the frequency band of the frequency component of the eddy current, the frequency component of the human body communication signal and the frequency component of the eddy current can be reliably separated from each other, and thereby, the signal components of the eddy current, which are noise components having a frequency band lower than that of the electric signals from the capsule endoscope, can be reliably eliminated. As a result, it is possible to realize a capsule guidance system in which the signal components (noise components) due to the eddy current generated in the subject when magnetically guiding the capsule endoscope inside the subject can be reliably eliminated, and the position of the capsule endoscope inside the subject can be detected and in-vivo images of the subject can be obtained while reliably preventing adverse effects caused by the eddy current.

Since the position of the capsule endoscope is calculated on the basis of the electrical signals from the capsule endoscope extracted by the digital filter, it is possible to continuously detect the position of the capsule endoscope inside the subject regardless of whether or not the eddy current is generated in the subject, and the capsule endoscope can be easily guided to a desired position inside the subject on the basis of the position detection result.

Further, since an in-vivo image of the subject is generated on the basis of the image signal demodulated from the electrical signal from the capsule endoscope extracted by the digital filter, it is possible to continuously obtain in-vivo images inside the subject regardless of whether or not the eddy current is generated in the subject. As a result, a greater amount of image data for observing the inside of organs of the subject can be obtained, so that it is possible to prevent a situation from occurring in which an in-vivo image of a portion of interest such as a bleeding site or a lesion site is missed.

Although, in the first embodiment of the invention, when the eddy current due to the magnetic field is generated in the subject 1, the signal components of the eddy current are eliminated by stopping the image generation processing of the image processing unit 7 and the position calculation processing of the position calculator 9, it is not limited to this. It may control the operation timing of the receiving unit 6 to stop the receiving processing of the receiving unit 6 when the eddy current due to the magnetic field is generated in the subject 1 and eliminate the signal components of the eddy current by such control. In this case, the control unit 14 controls the operation timing of the receiving unit 6 on the basis of whether or not the time rate of change of the magnetic field obtained from the magnetic field change detector 17 is smaller than or equal to a predetermined threshold value. Specifically, the control unit 14 determines whether or not the time rate of change of the magnetic field obtained from the magnetic field change detector 17 is smaller than or equal to a predetermined threshold value. If the time rate of change of the magnetic field exceeds the threshold value, the control unit 14 recognizes that the eddy current is generated in the subject 1 and causes the receiving unit 6 to stop the receiving processing, and if the time rate of change of the magnetic field is smaller than or equal to the threshold value, the control unit 14 recognizes that the eddy current is not generated in the subject 1 and causes the receiving unit 6 to perform the receiving processing.

Although, in the first to the fourth embodiments of the invention, a capsule guidance system that magnetically guides the capsule endoscope 2 having the imaging function for capturing in-vivo images in the subject and the human body communication function for transmitting the in-vivo images to the outside by using the human body as a communication medium is exemplified, it is not limited to this. The capsule medical device according to the invention may be a capsule type pH measuring device that measures pH in a living body, a capsule type drug dosing device having a function to disperse or inject a drug into a living body, or a capsule type collecting device that collects objects in a living body as long as the device has the human body communication function and can perform magnetic guidance.

Further, although, in the first to the fourth embodiments of the invention, the transmitting electrodes of the capsule endoscope 2 are realized by the transparent transmitting electrode 21a on the image capturing side and the opaque transmitting electrode 21b in the dome-shaped portion on the opposite side, it is not limited to this, and the arrangement and the pattern of the pair of transmitting electrodes can be arbitrarily determined. For example, a pair of transmitting electrodes may be provided on the helical protrusion 29, or it is possible to provide double helical protrusions and provide a transmitting electrode on each helical protrusion. In this way, the contact state between the capsule endoscope 2 and the subject 1 (a human body) can be made stable.

In order to improve the communication characteristics of the human body communication, it is possible to improve the contact state between the capsule endoscope 2 and the subject 1 by drinking ion water having impedance similar to that of the subject 1 when performing the examination. Further, as a method for guiding the capsule endoscope 2, the method in which the helical protrusion is rotated has been described, however, the invention is not limited to this, and the invention can also be applied to a method in which the capsule endoscope 2 is attracted and guided by a magnetic attraction force using magnetic gradient.

### Industrial Applicability

As described above, the capsule guidance system according to the present invention is suitable for detecting the position of the capsule medical device in the subject without being disturbed by the eddy current generated on the body surface when the capsule medical device in the subject is magnetically guided.

### Reference Signs List

- 1: SUBJECT

- 2: CAPSULE ENDOSCOPE
- 3, 33, 43, 53: RECEIVING DEVICE
- 4, 44: MAGNETIC GUIDANCE DEVICE
- 5: ELECTRODE PAD
- 6, 55: RECEIVING UNIT
- 7: IMAGE PROCESSING UNIT
- 8: ELECTRIC POTENTIAL DISTRIBUTION ACQUISITION UNIT
- 9: POSITION CALCULATOR
- 10, 30, 40, 50: CAPSULE GUIDANCE SYSTEM
- 11: INPUT UNIT
- 12: DISPLAY UNIT
- 13: STORAGE UNIT
- 14, 38, 48: CONTROL UNIT
- 15: MAGNETIC FIELD GENERATOR
- 16: SIGNAL GENERATOR
- 17: MAGNETIC FIELD CHANGE DETECTOR
- 18: MAGNETIC GUIDANCE CONTROLLER
- 20a: TUBULAR HOUSING
- 20b: DOME-SHAPED HOUSING
- 21a, 21b: TRANSMITTING ELECTRODE
- 22: ILLUMINATION UNIT
- 23: CONDENSER LENS
- 24: IMAGING ELEMENT
- 25: SIGNAL PROCESSING UNIT
- 26: BATTERY
- 27: TRANSMITTING UNIT
- 28: MAGNET
- 29: HELICAL PROTRUSION
- 36: EDDY CURRENT CALCULATOR
- 37: SUBTRACTION PROCESSING UNIT
- 49, 54: FILTER
- 55a: A/D CONVERTER
- 55b: FFT PROCESSING UNIT
- 55c: DEMODULATOR
- X1, X2, Y1, Y2, Z1, Z2: ELECTROMAGNET

## Claims

1. A capsule guidance system (10; 30; 40; 50), comprising:
a capsule medical device (2) adapted to be inserted into an organ of a subject (1) and comprising:
a magnet (28) disposed in the center of the capsule medical device (2);
transmitting electrodes (21 a, 21 b) formed respectively on the surfaces of opposing domes of a dome-shaped housing (20b) of the capsule medical device (2) to form an electric potential distribution on the body surface of the subject (1);
the capsule guidance system further comprising:
a plurality of electrode pads (5) adapted to be disposed on a body surface of the subject (1) and adapted to detect electrical signals transmitted by the transmitting electrodes (21a, 21b) from the capsule medical device (2) via the subject (1) used as a communication medium;
a magnetic guidance unit (15, 16, 18) adapted to apply a magnetic field to the capsule medical device (2) to guide the capsule medical device (2); and
a position calculation unit (9) adapted to calculate the position of the capsule medical device (2) based on the electrical signals from the capsule medical device (2), the capsule guidance system **characterized by**
an eliminating unit that is adapted to eliminate signal components of eddy current from the electrical signals detected by the plurality of electrode pads (5), wherein
the eddy current is generated in the subject (1) due to a change in the magnetic field, and
the electrical signals used by the position calculation unit (9) for calculating the position of the capsule medical device (2) are the electrical signals from which the signal components of the eddy current are eliminated by the eliminating unit.

2. The capsule guidance system (10) according to claim 1, wherein the eliminating unit includes
a magnetic field change detecting unit (17) that detects the time rate of change of the magnetic field applied to the capsule medical device (2) by the magnetic guidance unit (15, 16, 18), and
a control unit (14) that determines whether or not the time rate of change of the magnetic field exceeds a predetermined threshold value and stops position calculation processing of the capsule medical device (2) by the position calculation unit (9) when the time rate of change of the magnetic field exceeds the predetermined threshold value,
wherein the signal components of the eddy current are eliminated when the control unit (14) stops the position calculation processing of the capsule medical device (2).

3. The capsule guidance system (30) according to claim 1, wherein the eliminating unit includes
a magnetic field change detecting unit (17) that detects the time rate of change of the magnetic field applied to the capsule medical device (2) by the magnetic guidance unit (15, 16, 18),
an eddy current calculation unit (36) that calculates the signal components of the eddy current,
a subtraction processing unit (37) that performs subtraction processing for subtracting the signal components of the eddy current from voltages of the electrical signals detected by the plurality of electrode pads (5), and
a control unit (38) that determines whether or not the time rate of change of the magnetic field exceeds a predetermined threshold value and causes the subtraction processing unit (37) to perform the subtraction processing when the time rate of change of the magnetic field exceeds the predetermined threshold value,
wherein the position calculation unit (9) calculates the position of the capsule medical device (2) based on voltages of electrical signals from the capsule medical device (2), which are calculated by the subtraction processing of the subtraction processing unit (37).

4. The capsule guidance system (40) according to claim 1,
wherein the eliminating unit is a filter (49) for eliminating the signal components of the eddy current from the electrical signals detected by the plurality of electrode pads (5).

5. The capsule guidance system (50) according to claim 1, wherein the eliminating unit includes
a band-pass filter (54) for limiting the frequency band of the electrical signals detected by the plurality of electrode pads (5), and
a digital filter (55b) for converting the electrical signals whose frequency band is limited by the band-pass filter (54) into frequency components and eliminating a frequency component of the eddy current from the converted frequency components.

6. The capsule guidance system (10) according to claim 1, further comprising
a receiving unit (6) that receives electrical signals detected by the plurality of electrode pads (5), wherein
the eliminating unit includes
a magnetic field change detecting unit (17) that detects the time rate of change of the magnetic field applied to the capsule medical device (2) by the magnetic guidance unit (15, 16, 18), and
a control unit (14) that determines whether or not the time rate of change of the magnetic field exceeds a predetermined threshold value and controls the receiving unit (6) to eliminate the electrical signals when the time rate of change of the magnetic field exceeds the predetermined threshold value, and
the position calculation unit (9) calculates the position of the capsule medical device (2) based on electrical signals from the capsule medical device (2), which are outputted by the receiving unit (6).

7. The capsule guidance system (10, 30, 40, 50) according to any one of claims 1 to 6, further comprising
a data acquisition unit (7) that acquires data from the capsule medical device (2) based on the electrical signals from the capsule medical device (2), the electrical signals being the signals from which the signal components of the eddy current are eliminated by the eliminating unit.

8. The capsule guidance system (10, 30) according to claim 7,
wherein the eliminating unit eliminates the signal components of the eddy current by stopping data acquisition processing of the data acquisition unit (7) when the eddy current is generated in the subject (1).

## Patentansprüche

1. Kapselführungssystem (10; 30; 40; 50), das umfasst:
eine medizinische Kapselvorrichtung (2), die so ausgelegt ist, dass sie in ein Organ eines Individuums (1) eingesetzt wird, und die umfasst:
einen Magneten (28), der in der Mitte der medizinischen Kapselvorrichtung (2) angeordnet ist;
Sendeelektroden (21a, 21b), die jeweils auf den Oberflächen gegenüberliegender Kuppeln eines kuppelförmigen Gehäuses (20b) der medizinischen Kapselvorrichtung (2) gebildet sind, um eine Verteilung elektrischen Potentials auf der Körperoberfläche des Individuums (1) zu bilden;
wobei das Kapselführungssystem ferner umfasst:
eine Mehrzahl von Elektrodenkontaktstellen (5), die so ausgelegt sind, dass sie auf einer Körperfläche des Individuums (1) angeordnet werden, und die so ausgelegt sind, dass sie elektrische Signale erkennen, die durch die Sendeelektroden (21a, 21b) von der medizinischen Kapselvorrichtung (2) über das als Kommunikationsmedium verwendete Individuum (1) gesendet werden;
eine magnetische Führungseinheit (15, 16, 18), die so ausgelegt ist, dass sie ein Magnetfeld an die medizinische Kapselvorrichtung (2) anlegt, um die medizinische Kapselvorrichtung (2) zu führen; und
eine Positionsberechnungseinheit (9), die so ausgelegt ist, dass sie die Position der medizinischen Kapselvorrichtung (2) auf Basis der elektrischen Signale aus der medizinischen Kapselvorrichtung (2) berechnet,
wobei das Kapselführungssystem **gekennzeichnet ist durch**:
eine Eliminierungseinheit, die so ausgelegt ist, dass sie Signalkomponenten von Wirbelstrom aus den elektrischen Signalen eliminiert, die von der Mehrzahl von Elektrodenkontaktstellen (5) erkannt werden, wobei
der Wirbelstrom **durch** eine Änderung des Magnetfelds im Individuum (1) erzeugt wird, und
die elektrischen Signale, die von der Positionsberechnungseinheit (9) zum Berechnen der Position der medizinischen Kapselvorrichtung (2) verwendet werden, die elektrischen Signale sind, aus denen die Signalkomponenten des Wirbelstroms durch die Eliminierungseinheit eliminiert werden.

2. Kapselführungssystem (10) nach Anspruch 1, wobei die Eliminierungseinheit umfasst:
eine Magnetfeldänderungs-Erkennungseinheit (17), die die zeitliche Änderungsrate des Magnetfelds erkennt, das von der magnetischen Führungseinheit (15, 16, 18) an die medizinische Kapselvorrichtung (2) angelegt wird, und
eine Steuereinheit (14), die ermittelt, ob die zeitliche Änderungsrate des Magnetfelds einen vordefinierten Schwellenwert überschreitet, und die eine Positionsberechnungsverarbeitung der medizinischen Kapselvorrichtung (2) durch die Positionsberechnungseinheit (9) stoppt, wenn die zeitliche Änderungsrate des Magnetfelds den vordefinierten Schwellenwert überschreitet,
wobei die Signalkomponenten des Wirbelstroms eliminiert werden, wenn die Steuereinheit (14) die Positionsberechnungsverarbeitung der medizinischen Kapselvorrichtung (2) stoppt.

3. Kapselführungssystem (30) nach Anspruch 1, wobei die Eliminierungseinheit umfasst:
eine Magnetfeldänderungs-Erkennungseinheit (17), die die zeitliche Änderungsrate des Magnetfelds erkennt, das von der magnetischen Führungseinheit (15, 16, 18) an die medizinische Kapselvorrichtung (2) angelegt wird,
eine Wirbelstromberechnungseinheit (36), die die Signalkomponenten des Wirbelstroms berechnet,
eine Subtraktionsverarbeitungseinheit (37), die eine Subtraktionsverarbeitung zum Subtrahieren der Signalkomponenten des Wirbelstroms von Spannungen der elektrischen Signale, die von der Mehrzahl von Elektrodenkontaktstellen (5) erkannt werden, durchführt, und
eine Steuereinheit (38), die ermittelt, ob die zeitliche Änderungsrate des Magnetfelds einen vordefinierten Schwellenwert überschreitet, und die die Subtraktionsverarbeitungseinheit (37) dazu veranlasst, die Subtraktionsverarbeitung durchzuführen, wenn die zeitliche Änderungsrate des Magnetfelds den vordefinierten Schwellenwert überschreitet,
wobei die Positionsberechnungseinheit (9) die Position der medizinischen Kapselvorrichtung (2) auf Basis von Spannungen von elektrischen Signalen aus der medizinischen Kapselvorrichtung (2) berechnet, die durch die Subtraktionsverarbeitung der Subtraktionsverarbeitungseinheit (37) berechnet werden.

4. Kapselführungssystem (40) nach Anspruch 1,
wobei die Eliminierungseinheit ein Filter (49) zum Eliminieren der Signalkomponenten des Wirbelstroms aus den elektrischen Signalen ist, die von der Mehrzahl von Elektrodenkontaktstellen (5) erkannt werden.

5. Kapselführungssystem (50) nach Anspruch 1, wobei die Eliminierungseinheit umfasst:
ein Bandpassfilter (54) zum Begrenzen des Frequenzbandes der elektrischen Signale, die von der Mehrzahl von Elektrodenkontaktstellen (5) erkannt werden, und
ein digitales Filter (55b) zum Umwandeln der elektrischen Signale, deren Frequenzband durch das Bandpassfilter (54) begrenzt wird, in Frequenzkomponenten und zum Eliminieren einer Frequenzkomponente des Wirbelstroms aus den umgewandelten Frequenzkomponenten.

6. Kapselführungssystem (10) nach Anspruch 1, das ferner umfasst:
eine Empfangseinheit (6), die elektrische Signale empfängt, die von der Mehrzahl von Elektrodenkontaktstellen (5) erkannt werden, wobei
die Eliminierungseinheit umfasst:
eine Magnetfeldänderungs-Erkennungseinheit (17), die die zeitliche Änderungsrate des Magnetfelds erkennt, das von der magnetischen Führungseinheit (15, 16, 18) an die medizinische Kapselvorrichtung (2) angelegt wird,
eine Steuereinheit (14), die ermittelt, ob die zeitliche Änderungsrate des Magnetfelds einen vordefinierten Schwellenwert überschreitet, und die die Empfangseinheit (6) so steuert, dass sie die elektrischen Signale eliminiert, wenn die zeitliche Änderungsrate des Magnetfelds den vordefinierten Schwellenwert überschreitet, und
die Positionsberechnungseinheit (9) die Position der medizinischen Kapselvorrichtung (2) auf Basis von elektrischen Signalen aus der medizinischen Kapselvorrichtung (2) berechnet, die von der Empfangseinheit (6) ausgegeben werden.

7. Kapselführungssystem (10, 30, 40, 50) nach einem der Ansprüche 1 bis 6, das ferner umfasst:
eine Datenerfassungseinheit (7), die Daten aus der medizinischen Kapselvorrichtung (2) auf Basis der elektrischen Signale aus der medizinischen Kapselvorrichtung (2) erfasst, wobei die elektrischen Signale die Signale sind, aus denen die Signalkomponenten des Wirbelstroms durch die Eliminierungseinheit eliminiert werden.

8. Kapselführungssystem (10, 30) nach Anspruch 7,
wobei die Eliminierungseinheit die Signalkomponenten des Wirbelstroms durch Stoppen der Datenerfassungsverarbeitung der Datenerfassungseinheit (7) eliminiert, wenn der Wirbelstrom im Individuum (1) erzeugt wird.

## Revendications

1. Système de guidage de capsule (10 ; 30 ; 40 ; 50) comprenant :
un dispositif médical de capsule (2) adapté pour être introduit dans un organe d'un sujet (1) et comprenant :
un aimant (28) disposé au centre du dispositif médical de capsule (2) ;
des électrodes d'émission (21a, 21b) formées respectivement sur les surfaces de dômes opposés d'un boîtier en forme de dôme (20b) du dispositif médical de capsule (2) pour former une distribution de potentiel électrique sur la surface corporelle du sujet (1) ;
le système de guidage de capsule comprenant en outre :
une pluralité de pastilles d'électrode (5) adaptées pour être disposées sur une surface corporelle du sujet (1) et adaptées pour détecter des signaux électriques émis par les électrodes d'émission (21a, 21b) à partir du dispositif médical de capsule (2) par l'intermédiaire du sujet (1) utilisé comme support de communication ;
une unité de guidage magnétique (15, 16, 18) adaptée pour appliquer un champ magnétique au dispositif médical de capsule (2) pour guider le dispositif médical de capsule (2) ; et
une unité de calcul de position (9) adaptée pour calculer la position du dispositif médical de capsule (2) sur la base des signaux électriques provenant du dispositif médical de capsule (2),
le système de guidage de capsule étant **caractérisé par** une unité d'élimination qui est adaptée pour éliminer des composantes de signal de courant de Foucault à partir des signaux électriques détectés par la pluralité de pastilles d'électrode (5),
le courant de Foucault étant généré dans le sujet (1) en raison d'une variation dans le champ magnétique, et
les signaux électriques utilisés par l'unité de calcul de position (9) pour calculer la position du dispositif médical de capsule (2) étant les signaux électriques à partir desquels les composantes de signal du courant de Foucault sont éliminées par l'unité d'élimination.

2. Système de guidage de capsule (10) selon la revendication 1, dans lequel l'unité d'élimination comprend :
une unité de détection de variation de champ magnétique (17) qui détecte le taux de variation dans le temps du champ magnétique appliqué au dispositif médical de capsule (2) par l'unité de guidage magnétique (15, 16, 18), et
une unité de commande (14) qui détermine si le taux de variation dans le temps du champ magnétique dépasse ou non une valeur de seuil prédéterminée et arrête un traitement de calcul de position du dispositif médical de capsule (2) par l'unité de calcul de position (9) lorsque le taux de variation dans le temps du champ magnétique dépasse la valeur de seuil prédéterminée,
les composantes de signal du courant de Foucault étant éliminées lorsque l'unité de commande (14) arrête le traitement de calcul de position du dispositif médical de capsule (2).

3. Système de guidage de capsule (30) selon la revendication 1, dans lequel l'unité d'élimination comprend :
une unité de détection de variation de champ magnétique (17) qui détecte le taux de variation dans le temps du champ magnétique appliqué au dispositif médical de capsule (2) par l'unité de guidage magnétique (15, 16, 18),
une unité de calcul de courant de Foucault (36) qui calcule les composantes de signal du courant de Foucault,
une unité de traitement de soustraction (37) qui réalise un traitement de soustraction pour soustraire les composantes de signal du courant de Foucault aux tensions des signaux électriques détectés par la pluralité de pastilles d'électrode (5), et
une unité de commande (38) qui détermine si le taux de variation dans le temps du champ magnétique dépasse ou non une valeur de seuil prédéterminée et amène l'unité de traitement de soustraction (37) à réaliser le traitement de soustraction lorsque le taux de variation dans le temps du champ magnétique dépasse la valeur de seuil prédéterminée,
l'unité de calcul de position (9) calculant la position du dispositif médical de capsule (2) sur la base de tensions de signaux électriques provenant du dispositif médical de capsule (2), lesquelles sont calculées par le traitement de soustraction de l'unité de traitement de soustraction (37).

4. Système de guidage de capsule (40) selon la revendication 1, dans lequel l'unité d'élimination est un filtre (49) pour éliminer les composantes de signal du courant de Foucault à partir des signaux électriques détectés par la pluralité de pastilles d'électrode (5).

5. Système de guidage de capsule (50) selon la revendication 1, dans lequel l'unité d'élimination comprend :
un filtre passe-bande (54) pour limiter la bande de fréquence des signaux électriques détectés par la pluralité de pastilles d'électrode (5), et
un filtre numérique (55b) pour convertir les signaux électriques dont la bande de fréquence est limitée par le filtre passe-bande (54) en composantes de fréquence et éliminer une composante de fréquence du courant de Foucault à partir des composantes de fréquence converties.

6. Système de guidage de capsule (10) selon la revendication 1, comprenant en outre une unité de réception (6) qui reçoit des signaux électriques détectés par la pluralité de pastilles d'électrode (5),
l'unité d'élimination comprenant :
une unité de détection de variation de champ magnétique (17) qui détecte le taux de variation dans le temps du champ magnétique appliqué au dispositif médical de capsule (2) par l'unité de guidage magnétique (15, 16, 18), et
une unité de commande (14) qui détermine si le taux de variation dans le temps du champ magnétique dépasse ou non une valeur de seuil prédéterminée et commande l'unité de réception (6) pour éliminer les signaux électriques lorsque le taux de variation dans le temps du champ magnétique dépasse la valeur de seuil prédéterminée, et
l'unité de calcul de position (9) calcule la position du dispositif médical de capsule (2) sur la base de signaux électriques provenant du dispositif médical de capsule (2), lesquels sont délivrés en sortie par l'unité de réception (6).

7. Système de guidage de capsule (10, 30, 40, 50) selon l'une quelconque des revendications 1 à 6, comprenant en outre une unité d'acquisition de données (7) qui acquiert des données à partir du dispositif médical de capsule (2) sur la base des signaux électriques provenant du dispositif médical de capsule (2), les signaux électriques étant les signaux à partir desquels les composantes de signal du courant de Foucault sont éliminées par l'unité d'élimination.

8. Système de guidage de capsule (10, 30) selon la revendication 7, dans lequel l'unité d'élimination élimine les composantes de signal du courant de Foucault en arrêtant un traitement d'acquisition de données de l'unité d'acquisition de données (7) lorsque le courant de Foucault est généré dans le sujet (1).
